# EUROPEAN PATENT SPECIFICATION

(11) **EP 1 843 975 B1**
(45) Date of publication and mention of the grant of the patent: **11.06.2014**
(21) Application number: 05849889.0
(22) Date of filing: 02.11.2005
(51) Int. Cl.: C01B 39/12, C01B 39/48, C07C 2/12, C07C 29/48, C07C 45/28, C10G 11/05, C10G 45/64, C10G 47/16, C10G 50/00, C10G 29/20, C10G 3/00

(54) **MOLECULAR SIEVE SSZ-70 COMPOSITION OF MATTER AND SYNTHESIS THEREOF**
MOLSIEB-SSZ-70-STOFFZUSAMMENSETZUNG UND SYNTHESE DAVON
COMPOSITION DE MATIERE DE TAMIS MOLECULAIRE SSZ-70 ET SYNTHESE DE CELUI-CI

(30) Priority: 23.12.2004 US 639221 P; 23.12.2004 US 638475 P; 23.12.2004 US 639218 P; 23.12.2004 US 639212 P; 23.12.2004 US 639215 P
(43) Date of publication of application: 17.10.2007
(73) Proprietor: Chevron U.S.A. Inc., San Ramon, CA 94583 (US)
(72) Inventor: ZONES, Stacey, I., San Francisco, CA 94122 (US); BURTON, Allen, W., Jr., Richmond, CA 94804 (US)
(74) Representative: Nash, David Allan
(86) International application number: PCT/US2005/039648
(87) International publication number: WO 2006/071354

(56) References cited:
- EP-A- 0 522 196
- WO-A-96/29284
- WO-A-2004/060794
- US-A- 5 252 527
- US-A- 5 278 116
- US-A- 6 136 290

## Description

### BACKGROUND OF THE INVENTION

### Field of the Invention

The present invention relates to new crystalline molecular sieve SSZ-70, a method for preparing SSZ-70 using a N, N'-diisopropyl imidazolium cation as a structure directing agent and the use of SSZ-70 in catalysts for, e.g., hydrocarbon conversion reactions.

### State of the Art

Because of their unique sieving characteristics, as well as their catalytic properties, crystalline molecular sieves and zeolites are especially useful in applications such as hydrocarbon conversion, gas drying and separation. Although many different crystalline molecular sieves have been disclosed, there is a continuing need for new zeolites with desirable properties for gas separation and drying, hydrocarbon and chemical conversions, and other applications. New zeolites may contain novel internal pore architectures, providing enhanced selectivities in these processes.

Crystalline aluminosilicates are usually prepared from aqueous reaction mixtures containing alkali or alkaline earth metal oxides, silica, and alumina. Crystalline borosilicates are usually prepared under similar reaction conditions except that boron is used in place of aluminum. By varying the synthesis conditions and the composition of the reaction mixture, different zeolites can often be formed.

### SUMMARY OF THE INVENTION

The present invention is directed to a family of crystalline molecular sieves with unique properties, referred to herein as "molecular sieve SSZ-70" or simply "SSZ-70". Preferably, SSZ-70 is obtained in its silicate, aluminosilicate, titanosilicate, vanadosilicate or borosilicate form. The term "silicate" refers to a molecular sieve having a high mole ratio of silicon oxide relative to aluminum oxide, preferably a mole ratio greater than 100, including molecular sieves comprised entirely of silicon oxide. As used herein, the term "aluminosilicate" refers to a molecular sieve containing both aluminum oxide and silicon oxide and the term "borosilicate" refers to a molecular sieve containing oxides of both boron and silicon.

In accordance with this invention, there is provided a molecular sieve having a mole ratio greater than about 15 of (1) an oxide of a first tetravalent element to (2) an oxide of a trivalent element, pentavalent element, second tetravalent element different from said first tetravalent element or mixture thereof and having, after calcination, the X-ray diffraction lines of Table II.

Further, in accordance with this invention, there is provided a molecular sieve having a mole ratio greater than about 15 of (1) an oxide selected from silicon oxide, to (2) an oxide selected from aluminum oxide, gallium oxide, iron oxide, boron oxide, titanium oxide, vanadium oxide and mixtures thereof and having, after calcination, the X-ray diffraction lines of Table II below. It should be noted that the mole ratio of the first oxide or mixture of first oxides to the second oxide can be infinity, i.e., there is no second oxide in the molecular sieve. In these cases, the molecular sieve is an essentially all-silica molecular sieve.

The present invention further provides such a molecular sieve having a composition, as synthesized and in the anhydrous state, in terms of mole ratios as follows:

| | |
|---|---|
| YO₂/B₂O₃ | 20 - 60 |
| M_{2/n}/YO₂ | 0 - 0.03 |
| Q/YO₂ | 0.02 - 0.05 |
| F/YO₂ | 0 - 0.10 |

wherein Y is silicon; M is an alkali metal cation, alkaline earth metal cation or mixtures thereof; n is the valence of M (i.e., 1 or 2); F is fluorine and Q is a N, N'-diisopropyl imidazolium cation.

The present invention also enables a molecular sieve prepared by thermally treating a molecular sieve having a mole ratio of (1) silicon oxide to (2) an oxide selected from aluminum oxide, gallium oxide, iron oxide, boron oxide, titanium oxide, vanadium oxide and mixtures thereof greater than about 15 at a temperature of from about 200°C to about 800°C, the thus-prepared molecular sieve having the X-ray diffraction lines of Table II. The present invention also enables this thus-prepared molecular sieve which is predominantly in the hydrogen form, which hydrogen form is prepared by ion exchanging with an acid or with a solution of an ammonium salt followed by a second calcination. If the molecular sieve is synthesized with a high enough ratio of SDA cation to sodium ion, calcination alone may be sufficient. For high catalytic activity, the SSZ-70 molecular sieve should be predominantly in its hydrogen ion form. As used herein, "predominantly in the hydrogen form" means that, after calcination, at least 80% of the cation sites are occupied by hydrogen ions and/or rare earth ions.

Also enabled by the present invention is a method of preparing a crystalline material comprising (1) a first oxide comprising silicon oxide and (2) a second oxide comprising boron oxide and having a mole ratio of the first oxide to the second oxide greater than 15, said method comprising contacting under crystallization conditions sources of said oxides and a structure directing agent comprising a N, N'-diisopropyl imidazolium cation.

In accordance with the present invention there is provided a process for converting hydrocarbons comprising contacting a hydrocarbonaceous feed at hydrocarbon converting conditions with a catalyst comprising the molecular sieve of this invention. The molecular sieve may be predominantly in the hydrogen form. It may also be substantially free of acidity. The invention includes such a process wherein the molecular sieve has a mole ratio greater than about 15 of (1) silicon oxide to (2) an oxide selected from aluminum oxide, gallium oxide, iron oxide, boron oxide, titanium oxide, indium oxide and mixtures thereof, and has, after calcination, the X-ray diffraction lines of Table II.

Further provided by the present invention is a hydrocracking process comprising contacting a hydrocarbon feedstock under hydrocracking conditions with a catalyst comprising the molecular sieve of this invention, preferably predominantly in the hydrogen form.

This invention also includes a dewaxing process comprising contacting a hydrocarbon feedstock under dewaxing conditions with a catalyst comprising the molecular sieve of this invention, preferably predominantly in the hydrogen form.

The present invention also includes a process for improving the viscosity index of a dewaxed product of waxy hydrocarbon feeds comprising contacting the waxy hydrocarbon feed under isomerization dewaxing conditions with a catalyst comprising the molecular sieve of this invention, preferably predominantly in the hydrogen form.

The present invention further includes a process for producing a C₂₀₊ lube oil from a C₂₀₊ olefin feed comprising isomerizing said olefin feed under isomerization conditions over a catalyst comprising the molecular sieve of this invention. The molecular sieve may be predominantly in the hydrogen form. The catalyst may contain at least one Group VIII metal.

In accordance with this invention, there is also provided a process for catalytically dewaxing a hydrocarbon oil feedstock boiling above about 350°F (177°C) and containing straight chain and slightly branched chain hydrocarbons comprising contacting said hydrocarbon oil feedstock in the presence of added hydrogen gas at a hydrogen pressure of about 15-3000 psi (0.103 - 20.7 MPa) with a catalyst comprising the molecular sieve of this invention, preferably predominantly in the hydrogen form. The catalyst may contain at least one Group VIII metal. The catalyst may be a layered catalyst comprising a first layer comprising the molecular sieve of this invention, and a second layer comprising an aluminosilicate zeolite which is more shape selective than the molecular sieve of said first layer. The first layer may contain at least one Group VIII metal.

Also included in the present invention is a process for preparing a lubricating oil which comprises hydrocracking in a hydrocracking zone a hydrocarbonaceous feedstock to obtain an effluent comprising a hydrocracked oil, and catalytically dewaxing said effluent comprising hydrocracked oil at a temperature of at least about 400°F (204°C) and at a pressure of from about 15 psig to about 3000 psig (0.103 - 20.7 Mpa gauge)in the presence of added hydrogen gas with a catalyst comprising the molecular sieve of this invention. The molecular sieve may be predominantly in the hydrogen form. The catalyst may contain at least one Group VIII metal.

Further included in this invention is a process for isomerization dewaxing a raffinate comprising contacting said raffinate in the presence of added hydrogen with a catalyst comprising the molecular sieve of this invention. The raffinate may be bright stock, and the molecular sieve may be predominantly in the hydrogen form. The catalyst may contain at least one Group VIII metal.

Also included in this invention is a process for increasing the octane of a hydrocarbon feedstock to produce a product having an increased aromatics content comprising contacting a hydrocarbonaceous feedstock which comprises normal and slightly branched hydrocarbons having a boiling range above about 40°C and less than about 200°C, under aromatic conversion conditions with a catalyst comprising the molecular sieve of this invention made substantially free of acidity by neutralizing said molecular sieve with a basic metal. Also provided in this invention is such a process wherein the molecular sieve contains a Group VIII metal component.

Also provided by the present invention is a catalytic cracking process comprising contacting a hydrocarbon feedstock in a reaction zone under catalytic cracking conditions in the absence of added hydrogen with a catalyst comprising the molecular sieve of this invention, preferably predominantly in the hydrogen form. Also included in this invention is such a catalytic cracking process wherein the catalyst additionally comprises a large pore crystalline cracking component.

This invention further provides an isomerization process for isomerizing C₄ to C₇ hydrocarbons, comprising contacting a feed having normal and slightly branched C₄ to C₇ hydrocarbons under isomerizing conditions with a catalyst comprising the molecular sieve of this invention, preferably predominantly in the hydrogen form. The molecular sieve may be impregnated with at least one Group VIII metal, preferably platinum. The catalyst may be calcined in a steam/air mixture at an elevated temperature after impregnation of the Group VIII metal.

Also provided by the present invention is a process for alkylating an aromatic hydrocarbon which comprises contacting under alkylation conditions at least a molar excess of an aromatic hydrocarbon with a C₂ to C₂₀ olefin under at least partial liquid phase conditions and in the presence of a catalyst comprising the molecular sieve of this invention, preferably predominantly in the hydrogen form. The olefin may be a C₂ to C₄ olefin, and the aromatic hydrocarbon and olefin may be present in a molar ratio of about 4:1 to about 20:1, respectively. The aromatic hydrocarbon may be selected from the group consisting of benzene, toluene, ethylbenzene, xylene, naphthalene, naphthalene derivatives, dimethylnaphthalene or mixtures thereof.

Further provided in accordance with this invention is a process for transalkylating an aromatic hydrocarbon which comprises contacting under transalkylating conditions an aromatic hydrocarbon with a polyalkyl aromatic hydrocarbon under at least partial liquid phase conditions and in the presence of a catalyst comprising the molecular sieve of this invention, preferably predominantly in the hydrogen form. The aromatic hydrocarbon and the polyalkyl aromatic hydrocarbon may be present in a molar ratio of from about 1:1 to about 25:1, respectively.

The aromatic hydrocarbon may be selected from the group consisting of benzene, toluene, ethylbenzene, xylene, or mixtures thereof, and the polyalkyl aromatic hydrocarbon may be a dialkylbenzene.

Further provided by this invention is a process to convert paraffins to aromatics which comprises contacting paraffins under conditions which cause paraffins to convert to aromatics with a catalyst comprising the molecular sieve of this invention, said catalyst comprising gallium, zinc, or a compound of gallium or zinc.

In accordance with this invention there is also provided a process for isomerizing olefins comprising contacting said olefin under conditions which cause isomerization of the olefin with a catalyst comprising the molecular sieve of this invention

Further provided in accordance with this invention is a process for isomerizing an isomerization feed comprising an aromatic C₈ stream of xylene isomers or mixtures of xylene isomers and ethylbenzene, wherein a more nearly equilibrium ratio of ortho-, meta- and para-xylenes is obtained, said process comprising contacting said feed under isomerization conditions with a catalyst comprising the molecular sieve of this invention.

The present invention further provides a process for oligomerizing olefins comprising contacting an olefin feed under oligomerization conditions with a catalyst comprising the molecular sieve of this invention.

This invention also provides a process for converting oxygenated hydrocarbons comprising contacting said oxygenated hydrocarbon with a catalyst comprising the molecular sieve of this invention under conditions to produce liquid products. The oxygenated hydrocarbon may be a lower alcohol.

Further provided in accordance with the present invention is a process for the production of higher molecular weight hydrocarbons from lower molecular weight hydrocarbons comprising the steps of:
(a) introducing into a reaction zone a lower molecular weight hydrocarbon-containing gas and contacting said gas in said zone under C₂₊ hydrocarbon synthesis conditions with the catalyst and a metal or metal compound capable of converting the lower molecular weight hydrocarbon to a higher molecular weight hydrocarbon; and
(b) withdrawing from said reaction zone a higher molecular weight hydrocarbon-containing stream.

The present invention further provides a catalyst composition for promoting polymerization of 1-olefins, said composition comprising
(A) a molecular sieve having a mole ratio greater than about 15 of (1) an oxide of a first tetravalent element to (2) an oxide of a trivalent element, pentavalent element, second tetravalent element which is different from said first tetravalent element or mixture thereof and having, after calcination, the X-ray diffraction lines of Table II; and
(B) an organotitanium or organochromium compound.
The 1-olefin polymerization catalyst composition icludes compositions wherein oxide (1) is silicon oxide, and oxide (2) is an oxide selected from aluminum oxide, gallium oxide, iron oxide, boron oxide, titanium oxide, indium oxide.

The present invention further provides a process for polymerizing 1-olefins, which process comprises contacting 1-olefin monomer with a catalytically effective amount of a catalyst composition comprising
(A) a molecular sieve having a mole ratio greater than about 15 of (1) an oxide of a first tetravalent element to (2) an oxide of a trivalent element, pentavalent element, second tetravalent element which is different from said first tetravalent element or mixture thereof and having, after calcination, the X-ray diffraction lines of Table II;
   and
(B) an organotitanium or organochromium compound
under polymerization conditions which include a temperature and pressure suitable for initiating and promoting the polymerization reaction.
The polymerization process may employ a catalyst wherein oxide (1) is silicon oxide, and oxide (2) is an oxide selected from aluminum oxide, gallium oxide, iron oxide, boron oxide, titanium oxide, indium oxide. The 1-olefin monomer may be ethylene.

The present invention further provides a process for hydrogenating a hydrocarbon feed containing unsaturated hydrocarbons, the process comprising contacting the feed and hydrogen under conditions which cause hydrogenation with a catalyst comprising the molecular sieve of this invention. The catalyst can also contain metals, salts or complexes wherein the metal is selected from the group consisting of platinum, palladium, rhodium, iridium or combinations thereof, or the group consisting of nickel, molybdenum, cobalt, tungsten, titanium, chromium, vanadium, rhenium, manganese and combinations thereof.

In accordance with this invention, there is also provided a process for hydrotreating a hydrocarbon feedstock comprising contacting the feedstock with a hydrotreating catalyst and hydrogen under hydrotreating conditions, wherein the catalyst comprises the molecular sieve of this invention.

The present invention also enables a method for performing an acylation reaction on an aromatic substrate ArHₙ to form a product ArHₙ₋₁COR, the method comprising the steps of:
providing the aromatic substrate,
intimately mixing the substrate and an acylating agent, wherein the acylating agent is selected from the group consisting of a carboxylic acid derivative, a carboxylic acid, an acid anhydride, an ester, and an acyl halide, and
exposing an intimate mixture thus formed to a catalyst comprising a molecular sieve having a mole ratio greater than about 15 of (1) an oxide of a first tetravalent element to (2) an oxi de of a trivalent element, pentavalent element, second tetravalent element which is different from said first tetravalent element or mixture thereof and having, after calcination, the X-ray diffraction lines of Table II.

The present invention also enables a method for performing an acylation reaction on an aromatic substrate ArHₙ to form a product ArHₙ₋₁COR, the method comprising the steps of:
providing the aromatic substrate,
intimately mixing the substrate and an acylating agent, wherein the acylating agent is selected from the group consisting of a carboxylic acid derivative, a carboxylic acid, an acid anhydride, an ester, and an acyl halide, and
exposing an intimate mixture thus formed to a catalyst comprising a molecular sieve having a mole ratio greater than about 15 of (1) silicon oxide to (2) an oxide selected from aluminum oxide, gallium oxide, iron oxide, boron oxide, titanium oxide, indium oxide and mixtures thereof, and having, after calcination, the X-ray diffraction lines of Table II.

It should be noted that the mole ratio of the first oxide or mixture of first oxides to the second oxide can be infinity, i.e., there is no second oxide in the molecular sieve. In these cases, the molecular sieve is an essentially all-silica molecular sieve. The present invention also includes this thus-prepared molecular sieve which is predominantly in the hydrogen form, which hydrogen form is prepared by ion exchanging with an acid or with a solution of an ammonium salt followed by a second calcination. If the molecular sieve is synthesized with a high enough ratio of SDA cation to sodium ion, calcination alone may be sufficient. For high catalytic activity, the SSZ-70 molecular sieve may be predominantly in its hydrogen ion form. As used herein, "predominantly in the hydrogen form" means that, after calcination, at least 80% of the cation sites are occupied by hydrogen ions and/or rare earth ions.

In accordance with the present invention, there is provided a process for oxidation of hydrocarbons comprising contacting said hydrocarbon with an oxidizing agent in the presence of a catalytically effective amount of a crystalline, titanium-containing molecular sieve for a time and at a temperature effective to oxidize said hydrocarbon, wherein the crystalline titanium-containing molecular sieve is a molecular sieve having a mole ratio greater than about 15 of (1) silicon oxide to (2) titanium oxide, and having, after calcination, the X-ray diffraction lines of Table II.

There is further provided in accordance with this invention a process for epoxidation of an olefin comprising contacting said olefin with hydrogen peroxide in the presence of a catalytically effective amount of a crystalline, titanium-containing molecular sieve for a time and at a temperature effective to epoxidize said olefin, wherein the crystalline titanium-containing molecular sieve is a molecular sieve having a mole ratio greater than about 15 of (1) silicon oxide to (2) titanium oxide, and having, after calcination, the X-ray diffraction lines of Table II.

Further provided in accordance with the present invention is a process for oxidizing cyclohexane comprising contacting said cyclohexane with hydrogen peroxide in the presence of a catalytically effective amount of a crystalline, titanium-containing molecular sieve for a time and at a temperature effective to oxidize said cyclohexane, wherein the crystalline titanium-containing molecular sieve is a molecular sieve having a mole ratio greater than about 15 of (1) silicon oxide to (2) titanium oxide, and having, after calcination, the X-ray diffraction lines of Table II.

The present invention also provides a catalytic oxidation process comprising contacting under oxidation conditions (1) a reactant which is catalytically oxidizable in the presence of hydrogen peroxide, (2) aqueous hydrogen peroxide and (3) a catalytically effective amount of an oxidation catalyst comprising a molecular sieve having a mole ratio greater than about 15 of (1) silicon oxide to (2) titanium oxide, and having, after calcination, the X-ray diffraction lines of Table II.

The present invention also enables a process for the epoxidation of an olefin comprising contacting said olefin with hydrogen peroxide in the presence of a catalytically effective amount of a catalyst comprising a molecular sieve having a mole ratio greater than about 15 of (1) silicon oxide to (2) titanium oxide, and having, after calcination, the X-ray diffraction lines of Table II. In accordance with the present invention, there is provided a process for oxidation of hydrocarbons comprising contacting said hydrocarbon with an oxidizing agent in the presence of a catalytically effective amount of a crystalline, titanium-containing molecular sieve for a time and at a temperature effective to oxidize said hydrocarbon, wherein the crystalline titanium-containing molecular sieve is a molecular sieve having a mole ratio greater than about 15 of (1) silicon oxide to (2) titanium oxide, and having, after calcination, the X-ray diffraction lines of Table II.

There is further provided in accordance with this invention a process for epoxidation of an olefin comprising contacting said olefin with hydrogen peroxide in the presence of a catalytically effective amount of a crystalline, titanium-containing molecular sieve for a time and at a temperature effective to epoxidize said olefin, wherein the crystalline titanium-containing molecular sieve is a molecular sieve having a mole ratio greater than about 15 of (1) silicon oxide to (2) titanium oxide, and having, after calcination, the X-ray diffraction lines of Table II.

Further provided in accordance with the present invention is a process for oxidizing cyclohexane comprising contacting said cyclohexane with hydrogen peroxide in the presence of a catalytically effective amount of a crystalline, titanium-containing molecular sieve for a time and at a temperature effective to oxidize said cyclohexane, wherein the crystalline titanium-containing molecular sieve is a molecular sieve having a mole ratio greater than about 15 of (1) silicon oxide to (2) titanium oxide, and having after calcination, the X-ray diffraction lines of Table II.

The present invention also provides a catalytic oxidation process comprising contacting under oxidation conditions (1) a reactant which is catalytically oxidizable in the presence of hydrogen peroxide, (2) aqueous hydrogen peroxide and (3) a catalytically effective amount of an oxidation catalyst comprising a molecular sieve having a mole ratio greater than about 15 of (1) silicon oxide to (2) titanium oxide, and having, after calcination, the X-ray diffraction lines of Table II.

The present invention also enables a process for the epoxidation of an olefin comprising contacting said olefin with hydrogen peroxide in the presence of a catalytically effective amount of a catalyst comprising a molecular sieve having a mole ratio greater than about 15 of (1) silicon oxide to (2) titanium oxide, and having, after calcination, the X-ray diffraction lines of Table II.

### BRIEF DESCRIPTION OF THE DRAWINGS

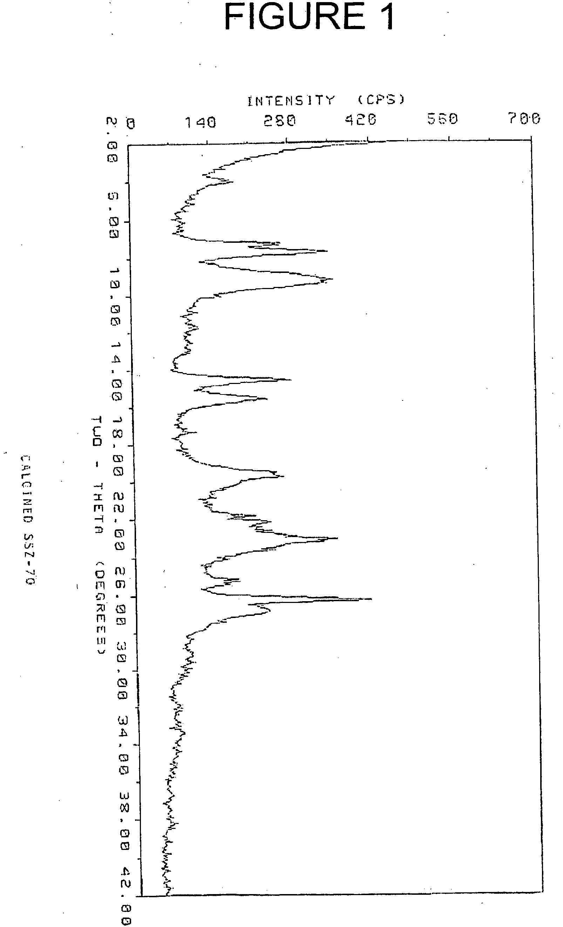

FIG. 1 is an X-ray diffraction pattern of SSZ-70 after it has been calcined.

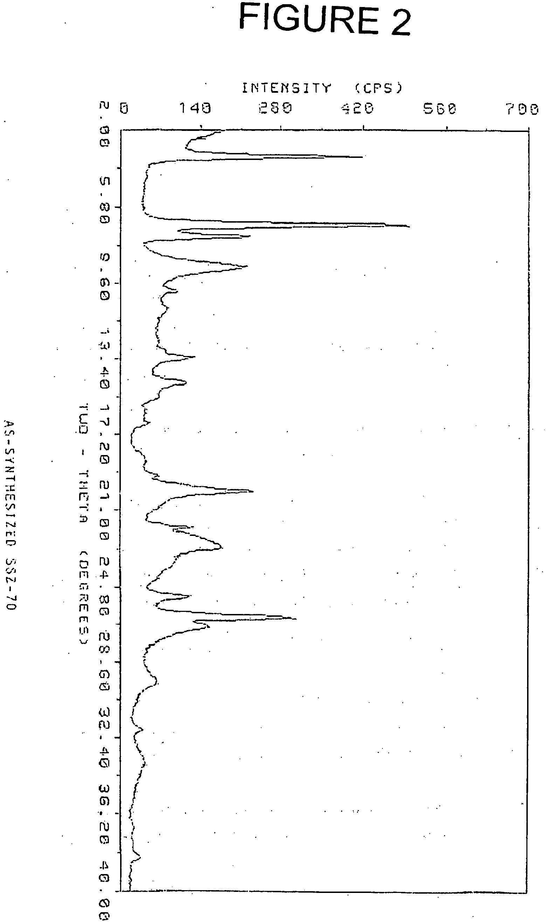

FIG. 2 is an X-ray diffraction pattern of SSZ- 70 in the as-synthesized form, i.e., prior to calcination with the SDA still in the pores of the SSZ-70.

### DETAILED DESCRIPTION OF THE INVENTION

The present invention comprises a family of crystalline molecular sieves designated herein "molecular sieve SSZ-70" or simply "SSZ-70". In preparing SSZ-70, a N, N'-diisopropyl imidazolium cation (referred to herein as "DIPI") is used as a structure directing agent ("SDA"), also known as a crystallization template. The SDA useful for making SSZ-70 has the following structure:

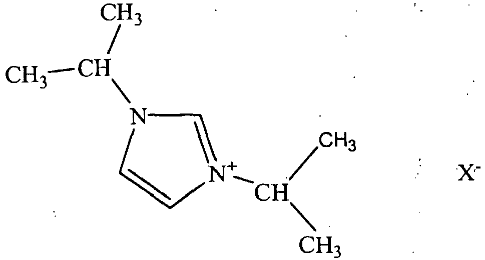

The SDA cation is associated with an anion (X⁻) which may be any anion that is not detrimental to the formation of the molecular sieve. Representative anions include halogen, e.g., fluoride, chloride, bromide and iodide, hydroxide, acetate, sulfate, tetrafluoroborate, carboxylate, and the like. Hydroxide is the most preferred anion.

SSZ-70 is prepared from a reaction mixture having the composition shown in Table A below.

**TABLE A**

| | Reaction Mixture | |
|---|---|---|
| | Typical | Preferred |
| YO₂/B₂O₃ | 5-60 | 10-50 |
| OH-/YO₂ | 0.10-0.50 | 0.20-0.30 |
| Q/YO₂ | 0.05-0.50 | 0.10-0.20 |
| M_{2/n}/YO₂ | 0-0.40 | 0.10-0.25 |
| H₂O/YO₂ | 30-80 | 35-45 |
| F/YO₂ | 0-0.50 | 0 |

where Y, Q, M, F and n are as defined above.

In practice, SSZ-70 is prepared by a process comprising:
(a) preparing an aqueous solution containing sources of at least two oxides capable of forming a crystalline molecular sieve and a DIPI cation having an anionic counterion which is not detrimental to the formation of SSZ-70;
(b) maintaining the aqueous solution under conditions sufficient to form crystals of SSZ-70; and
(c) recovering the crystals of SSZ-70.

Accordingly, SSZ-70 may comprise the crystalline material and the SDA in combination with metallic and non-metallic oxides bonded in tetrahedral coordination through shared oxygen atoms to form a cross-linked three dimensional crystal structure.

Typical sources of silicon oxide include silicates, silica hydrogel, silicic acid, fumed silica, colloidal silica, tetra-alkyl orthosilicates, and silica hydroxides. Boron can be added in forms corresponding to its silicon counterpart, such as boric acid.

A source zeolite reagent may provide a source of boron. In most cases, the source zeolite also provides a source of silica. The source zeolite in its deboronated form may also be used as a source of silica, with additional silicon added using, for example, the conventional sources listed above. Use of a source zeolite reagent for the present process is more completely described in U.S. Patent No. 5,225,179, issued July 6, 1993 to Nakagawa entitled "Method of Making Molecular Sieves".

Typically, an alkali metal hydroxide and/or an alkaline earth metal hydroxide, such as the hydroxide of sodium, potassium, lithium, cesium, rubidium, calcium, and magnesium, is used in the reaction mixture; however, this component can be omitted so long as the equivalent basicity is maintained. The SDA may be used to provide hydroxide ion. Thus, it may be benehcial to ion exchange, for example, the halide to hydroxide ion, thereby reducing or eliminating the alkali metal hydroxide quantity required. The alkali metal cation or alkaline earth cation may be part of the as-synthesized crystalline oxide material, in order to balance valence electron charges therein.

The reaction may also be carried out using HF to counterbalance the OH-contribution from the SDA, and run the synthesis in the absence of alkali cations. Running in the absence of alkali cations has the advantage of being able to prepare a catalyst from the synthesis product, by using calcination alone, i.e., no ion-exchange step (to remove alkali or alkaline earth cations) is necessary. In using HF, the reaction operates best when both the SDA and HF have mole ratios of 0.50 relative to YO₂ (e.g., silica).

The reaction mixture is maintained at an elevated temperature until the crystals of the SSZ-70 are formed. The hydrothermal crystallization is usually conducted under autogenous pressure, at a temperature between 100°C and 200°C, preferably between 135°C and 160°C. The crystallization period is typically greater than 1 day and preferably from about 3 days to about 20 days.

Preferably, the molecular sieve is prepared using mild stirring or agitation.

During the hydrothermal crystallization step, the SSZ-70 crystals can be allowed to nucleate spontaneously from the reaction mixture. The use of SSZ-70 crystals as seed material can be advantageous in decreasing the time necessary for complete crystallization to occur. In addition, seeding can lead to an increased purity of the product obtained by promoting the nucleation and/or formation of SSZ-70 over any undesired phases. When used as seeds, SSZ-70 crystals are added in an amount between 0.1 and 10% of the weight of first tetravalent element oxide, e.g. silica, used in the reaction mixture.

Once the molecular sieve crystals have formed, the solid product is separated from the reaction mixture by standard mechanical separation techniques such as filtration. The crystals are water-washed and then dried, e.g., at 90°C to 150°C for from 8 to 24 hours, to obtain the as-synthesized SSZ-70 crystals. The drying step can be performed at atmospheric pressure or under vacuum.

SSZ-70 as prepared has a mole ratio of silicon oxide to boron oxide greater than about 15; and has, after calcination, the X-ray diffraction lines of Table II below. SSZ-70 further has a composition, as synthesized (i.e., prior to removal of the SDA from the SSZ-70) and in the anhydrous state, in terms of mole ratios, shown in Table B below.

**TABLE B**

| As-Synthesized SSZ-70 | |
|---|---|
| YO₂/B₂O₃ | 20 - 60 |
| M_{2/n}/YO₂ | 0 - 0.03 |
| Q/YO₂ | 0.02 - 0.05 |
| F/YO₂ | 0 - 0.10 |

where Y, M, n, F and Q are as defined above.

SSZ-70 can be an essentiallyall-silica material. Thus, in a typical case where oxides of silicon and boron are used, SSZ-70 can be made essentially boron free, i.e., having a silica to boron oxide mole ratio of ∞. SSZ-70 is made as a borosilicate and then the boron can then be removed, if desired, by treating the borosilicate SSZ-70 with acetic acid at elevated temperature ( as described in Jones et al., Chem. Mater., 2001, 13, 1041-1050) to produce an all-silica version of SSZ-70.

If desired, SSZ-70 can be made as a borosilicate and then the boron can be removed as described above and replaced with metal atoms by techniques known in the art. Aluminum, gallium, iron, titanium, vanadium and mixtures thereof can be added in this manner.

It is believed that SSZ-70 is comprised of a new framework structure or topology which is characterized by its X-ray diffraction pattern. SSZ-70, as-synthesized, has a crystalline structure whose X-ray powder diffraction pattern exhibit the characteristic lines shown in Table I and is thereby distinguished from other molecular sieves.

**TABLE I**

| As-Synthesized SSZ-70 | | |
|---|---|---|
| 2 Theta^{(a)} | d-spacing (Angstroms) | Relative Intensity (%)^{(b)} |
| 3.32 | 26.6 | VS |
| 6.70 | 13.2 | VS |
| 7.26 | 12.2 | S |
| 8.78 | 10.1 | S |
| 13.34 | 6.64 | M |
| 20.02 | 4.44 | S |
| 22.54 | 3.94 | M |
| 22.88 | 3.89 | M |
| 26.36 | 3.38 | S-VS |
| 26.88 | 3.32 | M |

| | | |
|---|---|---|
| ^{(a)} ± 0.15 ^{(b)} The X-ray patterns provided are based on a relative intensity scale in which the strongest line in the X-ray pattern is assigned a value of 100: W(weak) is less than 20; M(medium) is between 20 and 40; S(strong) is between 40 and 60; VS(very strong) is greater than 60. | | |

Table IA below shows the X-ray powder diffraction lines for as-synthesized SSZ-70 including actual relative intensities.

**TABLE IA**

| 2 Theta^{(a)} | d-spacing (Angstroms) | Relative Intensity (%) |
|---|---|---|
| 3.32 | 26.6 | 84 |
| 6.70 | 13.2 | 100 |
| 7.26 | 12.2 | 45 |
| 8.78 | 10.1 | 44 |
| 13.34 | 6.64 | 26 |
| 20.02 | 4.44 | 46 |
| 22.54 | 3.94 | 33 |
| 22.88 | 3.89 | 36 |
| 26.36 | 3.38 | 61 |
| 26.88 | 3.32 | 31 |

| | | |
|---|---|---|
| ^{(a)} ± 0.15 | | |

After calcination, the SSZ-70 molecular sieves have a crystalline structure whose X-ray powder diffraction pattern include the characteristic lines shown in Table II:

**TABLE II**

| Calcined SSZ-70 | | |
|---|---|---|
| 2 Theta^{(a)} | d-spacing (Angstroms) | Relative Intensity (%) |
| 7.31 | 12.1 | VS |
| 7.75 | 11.4 | VS |
| 9.25 | 9.6 | VS |
| 14.56 | 6.08 | VS |
| 15.61 | 5.68 | S |
| 19.60 | 4.53 | S |
| 21.81 | 4.07 | M |
| 22.24 | 4.00 | M-S |
| 26.30 | 3.39 | VS |
| 26.81 | 3.33 | VS |

| | | |
|---|---|---|
| ^{(a)} ± 0.15 | | |

Table IIA below shows the X-ray powder diffraction lines for calcined SSZ-70 including actual relative intensities.

**TABLE IIA**

| 2 Theta^{(a)} | d-spacing (Angstroms) | Relative Intensity (%) |
|---|---|---|
| 7.31 | 12.1 | 67 |
| 7.75 | 11.4 | 93 |
| 9.25 | 9.6 | 79 |
| 14.56 | 6.08 | 68 |
| 15.61 | 5.68 | 49 |
| 19.60 | 4.53 | 58 |
| 21.81 | 4.07 | 38 |
| 22.24 | 4.00 | 41 |
| 26.30 | 3.39 | 99 |
| 26.81 | 3.33 | 80 |

| | | |
|---|---|---|
| ^{(a)} ±0.15 | | |

The X-ray powder diffraction patterns were determined by standard techniques. The radiation was the K-alpha/doublet of copper. The peak heights and the positions, as a function of 2θ where θ is the Bragg angle, were read from the relative intensities of the peaks, and d, the interplanar spacing in Angstroms corresponding to the recorded lines, can be calculated.

The variation in the scattering angle (two theta) measurements, due to instrument error and to differences between individual samples, is estimated at ± 0.15 degrees.

The X-ray diffraction pattern of Table I is representative of "as-synthesized" or "as-made" SSZ-70 molecular sieves. Minor variations in the diffraction pattern can result from variations in the silica-to-boron mole ratio of the particular sample due to changes in lattice constants. In addition, sufficiently small crystals will affect the shape and intensity of peaks, leading to significant peak broadening.

Representative peaks from the X-ray diffraction pattern of calcined SSZ-70 are shown in Table II. Calcination can also result in changes in the intensities of the peaks as compared to patterns of the "as-made" material, as well as minor shifts in the diffraction pattern. The molecular sieve produced by exchanging the metal or other cations present in the molecular sieve with various other cations (such as H⁺ or NH₄⁺) yields essentially the same diffraction pattern, although again, there may be minor shifts in the interplanar spacing and variations in the relative intensities of the peaks. Notwithstanding these minor perturbations, the basic crystal lattice remains unchanged by these treatments.

Crystalline SSZ-70 can be used as-synthesized, but preferably will be thermally treated (calcined). Usually, it is desirable to remove the alkali metal cation by ion exchange and replace it with hydrogen, ammonium, or any desired metal ion. The molecular sieve can be leached with chelating agents, e.g., EDTA or dilute acid solutions, to increase the silica to alumina mole ratio. The molecular sieve can also be steamed; steaming helps stabilize the crystalline lattice to attack from acids.

The molecular sieve can be used in intimate combination with hydrogenating components, such as tungsten, vanadium, molybdenum, rhenium, nickel, cobalt, chromium, manganese, or a noble metal, such as palladium or platinum, for those applications in which a hydrogenation-dehydrogenation function is desired.

Metals may also be introduced into the molecular sieve by replacing some of the cations in the molecular sieve with metal cations via standard ion exchange techniques (see, for example, U.S. Patent Nos. 3,140,249 issued July 7, 1964 to Plank et al.; 3,140,251 issued July 7, 1964 to Plank et al.; and 3,140,253 issued July 7, 1964 to Plank et al.). Typical replacing cations can include metal cations, e.g., rare earth, Group IA, Group IIA and Group VIII metals, as well as their mixtures. Of the replacing metallic cations, cations of metals such as rare earth, Mn, Ca, Mg, Zn, Cd, Pt, Pd, Ni, Co, Ti, Al, Sn, and Fe are particularly preferred.

The hydrogen, ammonium, and metal components can be ion-exchanged into the SSZ-70. The SSZ-70 can also be impregnated with the metals, or the metals can be physically and intimately admixed with the SSZ-70 using standard methods known to the art.

Typical ion-exchange techniques involve contacting the synthetic molecular sieve with a solution containing a salt of the desired replacing cation or cations. Although a wide variety of salts can be employed, chlorides and other halides, acetates, nitrates, and sulfates are particularly preferred. The molecular sieve is usually calcined prior to the ion-exchange procedure to remove the organic matter present in the channels and on the surface, since this results in a more effective ion exchange. Representative ion exchange techniques are disclosed in a wide variety of patents including U.S. Patent Nos. 3,140,249 issued on July 7, 1964 to Plank et al.; 3,140,251 issued on July 7, 1964 to Plank et al.; and 3,140,253 issued on July 7, 1964 to Plank et al.

Following contact with the salt solution of the desired replacing cation, the molecular sieve is typically washed with water and dried at temperatures ranging from 65°C to about 200°C. After washing, the molecular sieve can be calcined in air or inert gas at temperatures ranging from about 200°C to about 800°C for periods of time ranging from 1 to 48 hours, or more, to produce a catalytically active product especially useful in hydrocarbon conversion processes.

Regardless of the cations present in the synthesized form of SSZ-70, the spatial arrangement of the atoms which form the basic crystal lattice of the molecular sieve remains essentially unchanged.

SSZ-70 can be formed into a wide variety of physical shapes. Generally speaking, the molecular sieve can be in the form of a powder, a granule, or a molded product, such as extrudate having a particle size sufficient to pass through a 2-mesh (Tyler) screen and be retained on a 400-mesh (Tyler) screen. In cases where the catalyst is molded, such as by extrusion with an organic binder, the SSZ-70 can be extruded before drying, or, dried or partially dried and then extruded.

SSZ-70 can be composited with other materials resistant to the temperatures and other conditions employed in organic conversion processes. Such matrix materials include active and inactive materials and synthetic or naturally occurring zeolites as well as inorganic materials such as clays, silica and metal oxides. Examples of such materials and the manner in which they can be used are disclosed in U.S. Patent No. 4,910,006, issued May 20, 1990 to Zones et al., and U.S. Patent No. 5,316,753, issued May 31, 1994 to Nakagawa.

### Hydrocarbon Conversion Processes

SSZ-70 zeolites are useful in hydrocarbon conversion reactions. Hydrocarbon conversion reactions are chemical and catalytic processes in which carbon containing compounds are changed to different carbon containing compounds. Examples of hydrocarbon conversion reactions in which SSZ-70 are expected to be useful include hydrocracking, dewaxing, catalytic cracking and olefin and aromatics formation reactions. The catalysts are also expected to be useful in other petroleum refining and hydrocarbon conversion reactions such as isomerizing n-paraffins and naphthenes, polymerizing and oligomerizing olefinic or acetylenic compounds such as isobutylene and butene-1, polymerization of 1-olefins (e.g., ethylene), reforming, isomerizing polyalkyl substituted aromatics (e.g., m-xylene), and disproportionating aromatics (e.g., toluene) to provide mixtures of benzene, xylenes and higher methylbenzenes and oxidation reactions. Also included are rearrangement reactions to make various naphthalene derivatives, and forming higher molecular weight hydrocarbons from lower molecular weight hydrocarbons (e.g., methane upgrading).
The SSZ-70 catalysts may have high selectivity, and under hydrocarbon conversion conditions can provide a high percentage of desired products relative to total products.

For high catalytic activity, the SSZ-70 zeolite should be predominantly in its hydrogen ion form. Generally, the zeolite is converted to its hydrogen form by ammonium exchange followed by calcination. If the zeolite is synthesized with a high enough ratio of SDA cation to sodium ion, calcination alone may be sufficient. It is preferred that, after calcination, at least 80% of the cation sites are occupied by hydrogen ions and/or rare earth ions. As used herein, "predominantly in the hydrogen form" means that, after calcination, at least 80% of the cation sites are occupied by hydrogen ions and/or rare earth ions.

SSZ-70 zeolites can be used in processing hydrocarbonaceous feedstocks. Hydrocarbonaceous feedstocks contain carbon compounds and can be from many different sources, such as virgin petroleum fractions, recycle petroleum fractions, shale oil, liquefied coal, tar sand oil, synthetic paraffins from NAO, recycled plastic feedstocks and, in general, can be any carbon containing feedstock susceptible to zeolitic catalytic reactions. Depending on the type of processing the hydrocarbonaceous feed is to undergo, the feed can contain metal or be free of metals, it can also have high or low nitrogen or sulfur impurities. It can be appreciated, however, that in general processing will be more efficient (and the catalyst more active) the lower the metal, nitrogen, and sulfur content of the feedstock.

The conversion of hydrocarbonaceous feeds can take place in any convenient mode, for example, in fluidized bed, moving bed, or fixed bed reactors depending on the types of process desired. The formulation of the catalyst particles will vary depending on the conversion process and method of operation.

Other reactions which can be performed using the catalyst of this invention containing a metal, e.g., a Group VIII metal such platinum, include hydrogenation-dehydrogenation reactions, denitrogenation and desulfurization reactions.

The following table indicates typical reaction conditions which may be employed when using catalysts comprising SSZ-70 in the hydrocarbon conversion reactions of this invention. Preferred conditions are indicated in parentheses.

| Process | Temp.,°C | Pressure | LHSV |
|---|---|---|---|
| Hydrocracking | 175-485 | 0.5-350 bar | 0.1-30 |
| Dewaxing | 200-475 | 15-3000 psig, 0.103-20.7 Mpa gauge (200-3000, 1.38-20.7 Mpa gauge) | 0.1-20 |
| | (250-450) | | (0.2-10) |
| Aromatics formation | 400-600 | atom.-10 bar | 0.1-15 |
| | (480-550) | | |
| Cat. Cracking | 127-885 | subatm.⁻¹ (atm.-5 atm.) | 0.5-50 |
| Oligomerization | 232-649² | 0.1-50 atm.^{2,3} | 0.2-50² |
| | 10-232⁴ | - | 0.05-20⁵ |
| | (27-204)⁴ | - | (0.1-10)⁵ |
| Paraffins to aromatics | 100-700 | 0-1000 psig | 0.5-40⁵ |
| Condensation of alcohols | 260-538 | 0.5-1000 psig, 0.00345-6.89 Mpa gauge | 0.5-50⁵ |
| Isomerization | 93-538 | 50-1000 psig, 0.345-6.89 Mpa gauge | 1-10 |
| | (204-315) | | (1-4) |
| Xylene isomerization | 260-593² | 0.5-50 atm.² | 0.1-100⁵ |
| | (315-566)² | (1-5 atm)² | (0.5-50)⁵ |
| | 38-371⁴ | 1-200 atm.⁴ | 0.5-50 |

| | | | |
|---|---|---|---|
| ¹ Several hundred atmospheres ² Gas phase reaction ³ Hydrocarbon partial pressure ⁴ Liquid phase reaction ⁵ WHSV | | | |

Other reaction conditions and parameters are provided below.

### Hydrocracking

Using a catalyst which comprises SSZ-70, preferably predominantly in the hydrogen form, and a hydrogenation promoter, heavy petroleum residual feedstocks, cyclic stocks and other hydrocrackate charge stocks can be hydrocracked using the process conditions and catalyst components disclosed in the aforementioned U.S. Patent No. 4,910,006 and U.S. Patent No. 5,316,753.

The hydrocracking catalysts contain an effective amount of at least one hydrogenation component of the type commonly employed in hydrocracking catalysts. The hydrogenation component is generally selected from the group of hydrogenation catalysts consisting of one or more metals of Group VIB and Group VIII, including the salts, complexes and solutions containing such. The hydrogenation catalyst is preferably selected from the group of metals, salts and complexes thereof of the group consisting of at least one of platinum, palladium, rhodium, iridium, ruthenium and mixtures thereof or the group consisting of at least one of nickel, molybdenum, cobalt, tungsten, titanium, chromium and mixtures thereof. Reference to the catalytically active metal or metals is intended to encompass such metal or metals in the elemental state or in some form such as an oxide, sulfide, halide, carboxylate and the like. The hydrogenation catalyst is present in an effective amount to provide the hydrogenation function of the hydrocracking catalyst, and preferably in the range of from 0.05 to 25% by weight.

### Dewaxing

SSZ-70, preferably predominantly in the hydrogen form, can be used to dewax hydrocarbonaceous feeds by selectively removing straight chain paraffins. Typically, the viscosity index of the dewaxed product is improved (compared to the waxy feed) when the waxy feed is contacted with SSZ-70 under isomerization dewaxing conditions.

The catalytic dewaxing conditions are dependent in large measure on the feed used and upon the desired pour point. Hydrogen is preferably present in the reaction zone during the catalytic dewaxing process. The hydrogen to feed ratio is typically between about 500 and about 30,000 SCF/bbl (standard cubic feet per barrel) (0.089 to 5.34 SCM/liter (standard cubic meters/liter)), preferably about 1000 to about 20,000 SCF/bbl (0.178 to 3.56 SCM/liter). Generally, hydrogen will be separated from the product and recycled to the reaction zone. Typical feedstocks include light gas oil, heavy gas oils and reduced crudes boiling above about 350°F (177°C).

A typical dewaxing process is the catalytic dewaxing of a hydrocarbon oil feedstock boiling above about 350°F (177°C) and containing straight chain and slightly branched chain hydrocarbons by contacting the hydrocarbon oil feedstock in the presence of added hydrogen gas at a hydrogen pressure of about 15-3000 psi (0.103-20.7 Mpa) with a catalyst comprising SSZ-70 and at least one Group VIII metal.

The SSZ-70 hydrodewaxing catalyst may optionally contain a hydrogenation component of the type commonly employed in dewaxing catalysts. See the aforementioned U.S. Patent No. 4,910,006 and U.S. Patent No. 5,316,753 for examples of these hydrogenation components.

The hydrogenation component is present in an effective amount to provide an effective hydrodewaxing and hydroisomerization catalyst preferably in the range of from about 0.05 to 5% by weight. The catalyst may be run in such a mode to increase isomerization dewaxing at the expense of cracking reactions.

The feed may be hydrocracked, followed by dewaxing. This type of two stage process and typical hydrocracking conditions are described in U.S. Patent No. 4,921,594, issued May 1, 1990 to Miller.

SSZ-70 may also be utilized as a dewaxing catalyst in the form of a layered catalyst. That is, the catalyst comprises a first layer comprising zeolite SSZ-70 and at least one Group VIII metal, and a second layer comprising an aluminosilicate zeolite which is more shape selective than zeolite SSZ-70. The use of layered catalysts is disclosed in U.S. Patent No. 5,149,421, issued September 22, 1992 to Miller. The layering may also include a bed of SSZ-70 layered with a non-zeolitic component designed for either hydrocracking or hydrofinishing.

SSZ-70 may also be used to dewax raffinates, including bright stock, under conditions such as those disclosed in U. S. Patent No. 4,181,598, issued January 1, 1980 to Gillespie et al..

It is often desirable to use mild hydrogenation (sometimes referred to as hydrofinishing) to produce more stable dewaxed products. The hydrofinishing step can be performed either before or after the dewaxing step, and preferably after. Hydrofinishing is typically conducted at temperatures ranging from about 190°C to about 340°C at pressures from about 400 psig to about 3000 psig (2.76 to 20.7 Mpa gauge) at space velocities (LHSV) between about 0.1 and 20 and a hydrogen recycle rate of about 400 to 1500 SCF/bbl (0.071 to 0.27 SCM/liter). The hydrogenation catalyst employed must be active enough not only to hydrogenate the olefins, diolefins and color bodies which may be present, but also to reduce the aromatic content. Suitable hydrogenation catalyst are disclosed in U. S. Patent No. 4,921,594, issued May 1, 1990 to Miller. The hydrofinishing step is beneficial in preparing an acceptably stable product (e.g., a lubricating oil) since dewaxed products prepared from hydrocracked stocks tend to be unstable to air and light and tend to form sludges spontaneously and quickly.

Lube oil may be prepared using SSZ-70. For example, a C₂₀₊ lube oil may be made by isomerizing a C₂₀₊ olefin feed over a catalyst comprising SSZ-70 in the hydrogen form and at least one Group VIII metal. Alternatively, the lubricating oil, may be made by hydrocracking in a hydrocracking zone a hydrocarbonaceous feedstock to obtain an effluent comprising a hydrocracked oil, and catalytically dewaxing the effluent at a temperature of at least about 400°F (204°C) and at a pressure of from about 15 psig to about 3000 psig (0.103-20.7 Mpa gauge) in the presence of added hydrogen gas with a catalyst comprising SSZ-70 in the hydrogen form and at least one Group VIII metal.

### Aromatics Formation

SSZ-70 can be used to convert light straight run naphthas and similar mixtures to highly aromatic mixtures. Thus, normal and slightly branched chained hydrocarbons, preferably having a boiling range above about 40°C and less than about 200°C, can be converted to products having a substantial higher octane aromatics content by contacting the hydrocarbon feed with a catalyst comprising SSZ-70. It is also possible to convert heavier feeds into BTX or naphthalene derivatives of value using a catalyst comprising SSZ-70.

The conversion catalyst preferably contains a Group VIII metal compound to have sufficient activity for commercial use. By Group VIII metal compound as used herein is meant the metal itself or a compound thereof. The Group VIII noble metals and their compounds, platinum, palladium, and iridium, or combinations thereof can be used. Rhenium or tin or a mixture thereof may also be used in conjunction with the Group VIII metal compound and preferably a noble metal compound. The most preferred metal is platinum. The amount of Group VIII metal present in the conversion catalyst should be within the normal range of use in reforming catalysts, from about 0.05 to 2.0 weight percent, preferably 0.2 to 0.8 weight percent.

It is critical to the selective production of aromatics in useful quantities that the conversion catalyst be substantially free of acidity, for example, by neutralizing the zeolite with a basic metal, e.g., alkali metal, compound. Methods for rendering the catalyst free of acidity are known in the art. See the aforementioned U.S. Patent No. 4,910,006 and U.S. Patent No. 5,316,753 for a description of such methods.

The preferred alkali metals are sodium, potassium, rubidium and cesium. The zeolite itself can be substantially free of acidity only at very high silica:alumina mole ratios.

### Catalytic Cracking

Hydrocarbon cracking stocks can be catalytically cracked in the absence of hydrogen using SSZ-70, preferably predominantly in the hydrogen form.

When SSZ-70 is used as a catalytic cracking catalyst in the absence of hydrogen, the catalyst may be employed in conjunction with traditional cracking catalysts, e.g., any aluminosilicate heretofore employed as a component in cracking catalysts. Typically, these are large pore, crystalline aluminosilicates. Examples of these traditional cracking catalysts are disclosed in the aforementioned U.S. Patent No. 4,910,006 and U.S. Patent No 5,316,753. When a traditional cracking catalyst (TC) component is employed, the relative weight ratio of the TC to the SSZ-70 is generally between about 1:10 and about 500:1, desirably between about 1:10 and about 200:1, preferably between about 1:2 and about 50:1, and most preferably is between about 1:1 and about 20:1. The novel zeolite and/or the traditional cracking component may be further ion exchanged with rare earth ions to modify selectivity.

The cracking catalysts are typically employed with an inorganic oxide matrix component. See the aforementioned U.S. Patent No. 4,910,006 and U.S. Patent No. 5,316,753 for examples of such matrix components.

### Isomerization

The present catalyst is highly active and highly selective for isomerizing C₄ to C₇ hydrocarbons. The activity means that the catalyst can operate at relatively low temperature which thermodynamically favors highly branched paraffins. Consequently, the catalyst can produce a high octane product. The high selectivity means that a relatively high liquid yield can be achieved when the catalyst is run at a high octane.

The present process comprises contacting the isomerization catalyst, i.e., a catalyst comprising SSZ-70 in the hydrogen form, with a hydrocarbon feed under isomerization conditions. The feed is preferably a light straight run fraction, boiling within the range of 30°F to 250°F (-1°C to 121°C) and preferably from 60°F to 200°F (16°C to 93°C). Preferably, the hydrocarbon feed for the process comprises a substantial amount of C₄ to C₇ normal and slightly branched low octane hydrocarbons, more preferably C₅ and C₆ hydrocarbons.

It is preferable to carry out the isomerization reaction in the presence of hydrogen. Preferably, hydrogen is added to give a hydrogen to hydrocarbon ratio (H₂/HC) of between 0.5 and 10 H₂/HC, more preferably between 1 and 8 H₂/HC. See the aforementioned U.S. Patent No. 4,910,006 and U.S. Patent No. 5,316,753 for a further discussion of isomerization process conditions.

A low sulfur feed is especially preferred in the present process. The feed preferably contains less than 10 ppm, more preferably less than 1 ppm, and most preferably less than 0.1 ppm sulfur. In the case of a feed which is not already low in sulfur, acceptable levels can be reached by hydrogenating the feed in a presaturation zone with a hydrogenating catalyst which is resistant to sulfur poisoning. See the aforementioned U.S. Patent No. 4,910,006 and U.S. Patent No. 5,316,753 for a further discussion of this hydrodesulfurization process.

It is preferable to limit the nitrogen level and the water content of the feed. Catalysts and processes which are suitable for these purposes are known to those skilled in the art.

After a period of operation, the catalyst can become deactivated by sulfur or coke. See the aforementioned U.S. Patent No. 4,910,006 and U.S. Patent No. 5,316,753 for a further discussion of methods of removing this sulfur and coke, and of regenerating the catalyst.

The conversion catalyst preferably contains a Group VIII metal compound to have sufficient activity for commercial use. By Group VIII metal compound as used herein is meant the metal itself or a compound thereof. The Group VIII noble metals and their compounds, platinum, palladium, and iridium, or combinations thereof can be used. Rhenium and tin may also be used in conjunction with the noble metal. The most preferred metal is platinum. The amount of Group VIII metal present in the conversion catalyst should be within the normal range of use in isomerizing catalysts, from about 0.05 to 2.0 weight percent, preferably 0.2 to 0.8 weight percent.

### Alkylation and Transalkylation

SSZ-70 can be used in a process for the alkylation or transalkylation of an aromatic hydrocarbon. The process comprises contacting the aromatic hydrocarbon with a C₂ to C₁₆ olefin alkylating agent or a polyalkyl aromatic hydrocarbon transalkylating agent, under at least partial liquid phase conditions, and in the presence of a catalyst comprising SSZ-70.

SSZ-70 can also be used for removing benzene from gasoline by alkylating the benzene as described above and removing the alkylated product from the gasoline.

For high catalytic activity, the SSZ-70 zeolite should be predominantly in its hydrogen ion form. It is preferred that, after calcination, at least 80% of the cation sites are occupied by hydrogen ions and/or rare earth ions.

Examples of suitable aromatic hydrocarbon feedstocks which may be alkylated or transalkylated by the process of the invention include aromatic compounds such as benzene, toluene and xylene. The preferred aromatic hydrocarbon is benzene. There may be occasions where naphthalene or naphthalene derivatives such as dimethylnaphthalene may be desirable. Mixtures of aromatic hydrocarbons may also be employed.

Suitable olefins for the alkylation of the aromatic hydrocarbon are those containing 2 to 20, preferably 2 to 4, carbon atoms, such as ethylene, propylene, butene-1, trans-butene-2 and cis-butene-2, or mixtures thereof. There may be instances where pentenes are desirable. The preferred olefins are ethylene and propylene. Longer chain alpha olefins may be used as well.

When transalkylation is desired, the transalkylating agent is a polyalkyl aromatic hydrocarbon containing two or more alkyl groups that each may have from 2 to about: 4 carbon atoms. For example, suitable polyalkyl aromatic hydrocarbons include di-, tri-and tetra-alkyl aromatic hydrocarbons, such as diethylbenzene, triethylbenzene, diethylmethylbenzene (diethyltoluene), di-isopropylbenzene, di-isopropyltoluene, dibutylbenzene, and the like. Preferred polyalkyl aromatic hydrocarbons are the dialkyl benzenes. A particularly preferred polyalkyl aromatic hydrocarbon is di-isopropylbenzene.

When alkylation is the process conducted, reaction conditions are as follows. The aromatic hydrocarbon feed should be present in stoichiometric excess. It is preferred that molar ratio of aromatics to olefins be greater than four-to-one to prevent rapid catalyst fouling. The reaction temperature may range from 100°F to 600°F (38°C to 315°C), preferably 250°F to 450°F (121°C to 232°C). The reaction pressure should be sufficient to maintain at least a partial liquid phase in order to retard catalyst fouling. This is typically 50 psig to 1000 psig (0.345 to 6.89 Mpa gauge) depending on the feedstock and reaction temperature. Contact time may range from 10 seconds to 10 hours, but is usually from 5 minutes to an hour. The weight hourly space velocity (WHSV), in terms of grams (pounds) of aromatic hydrocarbon and olefin per gram (pound) of catalyst per hour, is generally within the range of about 0.5 to 50.

When transalkylation is the process conducted, the molar ratio of aromatic hydrocarbon will generally range from about 1:1 to 25:1, and preferably from about 2:1 to 20:1. The reaction temperature may range from about 100°F to 600°F (38°C to 315°C), but it is preferably about 250°F to 450°F (121°C to 232°C). The reaction pressure should be sufficient to maintain at least a partial liquid phase, typically in the range of about 50 psig to 1000 psig (0.345 to 6.89 Mpa gauge), preferably 300 psig to 600 psig (2.07 to 4.14 Mpa gauge). The weight hourly space velocity will range from about 0.1 to 10. U.S. Patent No. 5,082,990 issued on January 21, 1992 to Hsieh, et al. describes such processes.

### Conversion of Paraffins to Aromatics

SSZ-70 can be used to convert light gas C₂-C₆ paraffins to higher molecular weight hydrocarbons including aromatic compounds. Preferably, the zeolite will contain a catalyst metal or metal oxide wherein said metal is selected from the group consisting of Groups IB, IIB, VIII and IIIA of the Periodic Table. Preferably, the metal is gallium, niobium, indium or zinc in the range of from about 0.05 to 5% by weight.

### Isomerization of Olefins

SSZ-70 can be used to isomerize olefins. The feed stream is a hydrocarbon stream containing at least one C₄₋₆ olefin, preferably a C₄₋₆ normal olefin, more preferably normal butene. Normal butene as used in this specification means all forms of normal butene, e.g., 1-butene, cis-2-butene, and trans-2-butene. Typically, hydrocarbons other than normal butene or other C₄₋₆ normal olefins will be present in the feed stream. These other hydrocarbons may include, e.g., alkanes, other olefins, aromatics, hydrogen, and inert gases.

The feed stream typically may be the effluent from a fluid catalytic cracking unit or a methyl-tert-butyl ether unit. A fluid catalytic cracking unit effluent typically contains about 40-60 weight percent normal butenes. A methyl-tert-butyl ether unit effluent typically contains 40-100 weight percent normal butene. The feed stream preferably contains at least about 40 weight percent normal butene, more preferably at least about 65 weight percent normal butene. The terms iso-olefin and methyl branched iso-olefin may be used interchangeably in this specification.

The process is carried out under isomerization conditions. The hydrocarbon feed is contacted in a vapor phase with a catalyst comprising the SSZ-70. The process may be carried out generally at a temperature from about 625°F to about 950°F (329-510°C), for butenes, preferably from about 700°F to about 900°F (371-482°C), and about 350°F to about 650°F(177-343°C) for pentenes and hexenes. The pressure ranges from subatmospheric to about 200 psig (1.38 Mpa gauge), preferably from about 15 psig to about 200 psig (0.103 to 1.38 Mpa gauge), and more preferably from about 1 psig to about 150 psig (0.00689 to 1.03 Mpa gauge).

The liquid hourly space velocity during contacting is generally from about 0.1 to about 50 hr⁻¹, based on the hydrocarbon feed, preferably from about 0.1 to about 20 hr⁻¹, more preferably from about 0.2 to about 10 hr⁻¹, most preferably from about 1 to about 5 hr⁻¹. A hydrogen/hydrocarbon molar ratio is maintained from about 0 to about 30 or higher. The hydrogen can be added directly to the feed stream or directly to the isomerization zone. The reaction is preferably substantially free of water, typically less than about two weight percent based on the feed. The process can be carried out in a packed bed reactor, a fixed bed, fluidized bed reactor, or a moving bed reactor. The bed of the catalyst can move upward or downward. The mole percent conversion of, e.g., normal butene to iso-butene is at least 10, preferably at least 25, and more preferably at least 35.

### Xylene Isomerization

SSZ-70 may also be useful in a process for isomerizing one or more xylene isomers in a C₈ aromatic feed to obtain ortho-, meta-, and para-xylene in a ratio approaching the equilibrium value. In particular, xylene isomerization is used in conjunction with a separate process to manufacture para-xylene. For example, a portion of the para-xylene in a mixed C₈ aromatics stream may be recovered by crystallization and centrifugation. The mother liquor from the crystallizer is then reacted under xylene isomerization conditions to restore ortho-, meta- and para-xylenes to a near equilibrium ratio. At the same time, part of the ethylbenzene in the mother liquor is converted to xylenes or to products which are easily separated by filtration. The isomerate is blended with fresh feed and the combined stream is distilled to remove heavy and light byproducts. The resultant C₈ aromatics stream is then sent to the crystallizer to repeat the cycle.

Optionally, isomerization in the vapor phase is conducted in the presence of 3.0 to 30.0 moles of hydrogen per mole of alkylbenzene (e.g., ethylbenzene). If hydrogen is used, the catalyst should comprise about 0.1 to 2.0 wt.% of a hydrogenation/dehydrogenation component selected from Group VIII (of the Periodic Table) metal component, especially platinum or nickel. By Group VIII metal component is meant the metals and their compounds such as oxides and sulfides.

Optionally, the isomerization feed may contain 10 to 90 wt. of a diluent such as toluene, trimethylbenzene, naphthenes or paraffins.

### Oligomerization

It is expected that SSZ-70 can also be used to oligomerize straight and branched chain olefins having from about 2 to 21 and preferably 2-5 carbon atoms. The oligomers which are the products of the process are medium to heavy olefins which are useful for both fuels, i,e., gasoline or a gasoline blending stock and chemicals.

The oligomerization process comprises contacting the olefin feedstock in the gaseous or liquid phase with a catalyst comprising SSZ-70.

The zeolite can have the original cations associated therewith replaced by a wide variety of other cations according to techniques well known in the art. Typical cations would include hydrogen, ammonium and metal cations including mixtures of the same. Of the replacing metallic cations, particular preference is given to cations of metals such as rare earth metals, manganese, calcium, as well as metals of Group II of the Periodic Table, e.g., zinc, and Group VIII of the Periodic Table, e.g., nickel. One of the prime requisites is that the zeolite have a fairly low aromatization activity, i.e., in which the amount of aromatics produced is not more than about 20% by weight. This is accomplished by using a zeolite with controlled acid activity [alpha value] of from about 0,1 to about 120, preferably from about 0.1 to about 100, as measured by its ability to crack n-hexane.

Alpha values are defined by a standard test known in the art, e.g., as shown in U.S. Patent No. 3,960,978 issued on June 1, 1976 to Givens et al. If required, such zeolites may be obtained by steaming, by use in a conversion process or by any other method which may occur to one skilled in this art.

### Condensation of Alcohols

SSZ-70 can be used to condense lower aliphatic alcohols having 1 to 10 carbon atoms to a gasoline boiling point hydrocarbon product comprising mixed aliphatic and aromatic hydrocarbon. The process disclosed in U.S. Patent No. 3,894,107, issued July 8, 1975 to Butter et al., describes the process conditions used in this process.

The catalyst may be in the hydrogen form or may be base exchanged or impregnated to contain ammonium or a metal cation complement, preferably in the range of from about 0.05 to 5% by weight. The metal cations that may be present include any of the metals of the Groups I through VIII of the Periodic Table. However, in the case of Group IA metals, the cation content should in no case be so large as to effectively inactivate the catalyst, nor should the exchange be such as to eliminate all acidity. There may be other processes involving treatment of oxygenated substrates where a basic catalyst is desired.

### Methane Upgrading

Higher molecular weight hydrocarbons can be formed from lower molecular weight hydrocarbons by contacting the lower molecular weight hydrocarbon with a catalyst comprising SSZ-70 and a metal or metal compound capable of converting the lower molecular weight hydrocarbon to a higher molecular weight hydrocarbon. Examples of such reactions include the conversion of methane to C₂₊ hydrocarbons such as ethylene or benzene or both. Examples of useful metals and metal compounds include lanthanide and or actinide metals or metal compounds.

These reactions, the metals or metal compounds employed and the conditions under which they can be run are disclosed in U.S. Patents No. 4,734,537, issued March 29, 1988 to Devries et al.; 4,939,311, issued July 3, 1990 to Washecheck et al.; 4,962,261, issued October 9, 1990 to Abrevaya et al.; 5,095,161, issued March 10, 1992 to Abrevaya et al.; 5,105,044, issued April 14, 1992 to Han et al.; 5,105,046, issued April 14, 1992 to Washecheck; 5,238,898, issued August 24, 1993 to Han et al.; 5,321,185, issued June 14, 1994 to van der Vaart; and 5,336,825, issued August 9, 1994 to Choudhary et al.

### Polymerization of 1-Olefins

The molecular sieve of the present invention may be used in a catalyst for the polymerization of 1-olefins, e.g., the polymerization of ethylene. To form the olefin polymerization catalyst, the molecular sieve as hereinbefore described is reacted with a particular type of organometallic compound. Organometallic compounds useful in forming the polymerization catalyst include trivalent and tetravalent organotitanium and organochromium compounds having alkyl moieties and, optionally, halo moieties. In the context of the present invention the term "alkyl" includes both straight and branched chain alkyl, cycloalkyl and alkaryl groups such as benzyl.

Examples of trivalent and tetravalent organochromium and organotitanium compounds are disclosed in U. S. Patent No. 4,376,722, issued March 15, 1983 to Chester et al., U. S. Patent No. 4,377,497, issued March 22, 1983 to Chester et al., U. S. Patent No. 4,446,243, issued May 1, 1984 to Chester et al., and U. S. Patent No. 4,526,942, issued July 2, 1985 to Chester et al.

Examples of the organometallic compounds used to form the polymerization catalyst include, but are not limited to, compounds corresponding to the general formula:

MYₙXₘ₋ₙ

wherein M is a metal selected from titanium and chromium; Y is alkyl; X is halogen (e.g., Cl or Br); n is 1-4; and m is greater than or equal to n and is 3 or 4.

Examples of organotitanium and organochromium compounds encompassed by such a formula include compounds of the formula CrY₄, CrY₃, CrY₃X, CrY₂X, CrY₂X₂, CrYX₂, CrYX₃, TiY₄, TiY₃, TiY₃X, TiY₂X, TiY₂X₂, TiYX₂, TiYX₃, wherein X can be Cl or Br and Y can be methyl, ethyl, propyl, isopropyl, butyl, isobutyl, sec-butyl, tert-butyl, pentyl, isopentyl, neopentyl, hexyl, isohexyl, neohexyl, 2-ethybutyl, octyl, 2-ethylhexyl, 2,2-diethylbutyl, 2-isopropyl-3-methylbutyl, etc., cyclohexylalkyls such as, for example, cyclohexylmethyl, 2-cyolohexylethyl, 3-cyclyhexylpropyl, 4-cyclohexylbutyl, and the corresponding alkyl-substituted cyclohexyl radicals as, for example, (4-methylcyclohexyl)methyl, neophyl, i.e., beta, beta-dimethyl-phenethyl, benzyl, ethylbenzyl, and p-isopropylbenzyl. Preferred examples of Y include C₁₋₅ alkyl, especially butyl.

The organotitanium and organochromium materials employed in the catalyst can be prepared by techniques well known in the art. See, for examples the aforementioned Chester et al. patents.

The organotitanium or organochromium compounds can be with the molecular sieve of the present invention, such as by reacting the organometallic compound and the molecular sieve, in order to form the olefin polymerization catalyst. Generally, such a reaction takes place in the same reaction medium used to prepare the organometallic compound under conditions which promote formation of such a reaction product. The molecular sieve can simply be added to the reaction mixture after formation of the organometallic compound has been completed. Molecular sieve is added in an amount sufficient to provide from about 0.1 to 10 parts by weight, preferably from about 0.5 to 5 parts by weight, of organometallic compound in the reaction medium per 100 parts by weight of molecular sieve.

Temperature of the reaction medium during reaction of organometallic compound with molecular sieve is also maintained at a level which is low enough to ensure the stability of the organometallic reactant. Thus, temperatures in the range of from about - 150°, C. to 50° C., preferably from about -80° C. to 0° C. can be usefully employed. Reaction times of from about 0.01 to 10 hours, more preferably from about 0.1 to 1 hour, can be employed in reacting the organotitanium or organochromium compound with the molecular sieve.

Upon completion of the reaction, the catalyst material so formed may be recovered and dried by evaporating the reaction medium solvent under a nitrogen atmosphere. Alternatively, olefin polymerization reactions can be conducted in this same solvent based reaction medium used to form the catalyst.

The polymerization catalyst can be used to catalyze polymerization of 1-olefins. The polymers produced using the catalysts of this invention are normally solid polymers of at least one mono-1-olefin containing from 2 to 8 carbon atoms per molecule. These polymers are normally solid homopolymers of ethylene or copolymers of ethylene with another mono-1-olefin containing 3 to 8 carbon atoms per molecule. Exemplary copolymers include those of ethylene/propylene, ethylene/1-butene, ethylene/1-hexane, and ethylene/1-octene and the like. The major portion of such copolymers is derived from ethylene and generally consists of about 80-99, preferably 95-99 mole percent of ethylene. These polymers are well suited for extrusion, blow molding, injection molding and the like.

The polymerization reaction can be conducted by contacting monomer or monomers, e.g., ethylene, alone or with one or more other olefins, and in the substantial absence of catalyst poisons such as moisture and air, with a catalytic amount of the supported organometallic catalyst at a temperature and at a pressure sufficient to initiate the polymerization reaction. If desired, an inert organic solvent may be used as a diluent and to facilitate materials handling if the polymerization reaction is conducted with the reactants in the liquid phase, e.g. in a particle form (slurry) or solution process. The reaction may also be conducted with reactants in the vapor phase, e.g., in a fluidized bed arrangement in the absence of a solvent but, if desired, in the presence of an inert gas such as nitrogen.

The polymerization reaction is carried out at temperatures of from about 30° C. or less, up to about 200° C. or more, depending to a great extent on the operating pressure, the pressure of the olefin monomers, and the particular catalyst being used and its concentration. Naturally, the selected operating temperature is also dependent upon the desired polymer melt index since temperature is definitely a factor in adjusting the molecular weight of the polymer. Preferably, the temperature used is from about 30° C. to about 100° C. in a conventional slurry or "particle forming" process or from 100° C. to 150° C. in a "solution forming" process. A temperature of from about 70° C to 110° C. can be employed for fluidized bed processes.

The pressure to be used in the polymerization reactions can be any pressure sufficient to initiate the polymerization of the monomer(s) to high molecular weight polymer. The pressure, therefore, can range from subatmospheric pressures, using an inert gas as diluent, to superatmospheric pressures of up to about 30,000 psig or more. The preferred pressure is from atmospheric (0 psig) up to about 1000 psig. As a general rule, a pressure of 20 to 800 psig is most preferred.

The selection of an inert organic solvent medium to be employed in the solution or slurry process embodiments of this invention is not too critical, but the solvent should be inert to the supported organometallic catalyst and olefin polymer produced, and be stable at the reaction temperature used. It is not necessary, however, that the inert organic solvent medium also serve as a solvent for the polymer to be produced. Among the inert organic solvents applicable for such purposes may be mentioned saturated aliphatic hydrocarbons having from about 3 to 12 carbon atoms per molecule such as hexane, heptane, pentane, isooctane, purified kerosene and the like, saturated cycloaliphatic hydrocarbons having from about 5 to 12 carbon atoms per molecule such as cyclohexane, cyclopentane, dimethylcyclopentane and methylcyclohexane and the like and aromatic hydrocarbons having from about 6 to 12 carbon atoms per molecule such as benzene, toluene, xylene, and the like. Particularly preferred solvent media are cyclohexane, pentane, hexane and heptane.

Hydrogen can be introduced into the polymerization reaction zone in order to decrease the molecular weight of the polymers produced (i.e., give a much higher Melt Index, MI). Partial pressure of hydrogen when hydrogen is used can be within the range of 5 to 100 psig, preferably 25 to 75 psig. The melt indices of the polymers produced in accordance with the instant invention can range from about 0.1 to about 70 or even higher.

More detailed description of suitable polymerization conditions including examples of particle form, solution and fluidized bed polymerization arrangements are found in Karapinka; U.S. Pat No, 3,709,853; Issued Jan. 9, 1973 and Karol et al; U.S. Pat. No. 4,086,408; Issued Apr. 25, 1978.

### Hydrotreating

SSZ-70 is useful in a hydrotreating catalyst. During hydrotreatment, oxygen, sulfur and nitrogen present in the hydrocarbonaceous feed is reduced to low levels. Aromatics and olefins, if present in the feed, may also have their double bonds saturated. In some cases, the hydrotreating catalyst and hydrotreating conditions are selected to minimize cracking reactions, which can reduce the yield of the most desulfdedproduct (typically useful as a fuel).

Hydrotreating conditions typically include a reaction temperature between 400-900°F (204-482°C), preferably 650-850°F (343-454°C); a pressure between 500 and 5000 psig (3.5-34.6 Mpa), preferably 1000 to 3000 psig (7.0-20.8 MPa); a feed rate (LHSV) of 0.5 hr⁻¹ to 20 hr⁻¹ (v/v); and overall hydrogen consumption 300 to 2000 scf per barrel of liquid hydrocarbon feed (53.4-356 m³ H₂/m³ feed). The hydrotreating catalyst will typically be a composite of a Group VI metal or compound thereof, and a Group VIII metal or compound thereof supported on the molecular sieve of this invention.. Typically, such hydrotreating catalyst are presulfided.

Catalysts useful for hydrotreating hydrocarbon feeds are disclosed in U. S. Patents No. 4,347,121, issued August 31,1982 to Mayer et al, and 4,810,357, issued March 7, 1989 to Chester et al. Suitable catalysts include noble metals from Group VIII, such as Fe, Co, Ni, Pt or Pd, and/or Group VI metals, such as Cr, Mo, Sn or W. Examples of combinations of Group VIII and Group VI metals include Ni-Mo or Ni-Sn. Other suitable catalysts are described in U. S. Patents No. 4,157,294, issued June 5, 1979 to Iwao et al, and 3,904,513; issued September 9, 1975 to Fischer et al. U. S. Patent No. 3,852,207, issued December 3, 1974 to Strangeland et al, describes suitable noble metal catalysts and mild hydrotreating conditions.

The amount of hydrogenation component(s) in the catalyst suitably range from about 0.5% to about 10% by weight of Group VIII component(s) and from 5% to about 25% by weight of Group VI metal component(s), calculated as metal oxide(s) per 100 parts by weight of total catalyst., where the percentages by weight are based on the weight of the catalyst before sulfiding. The hydrogenation component(s) in the catalyst may be in the oxidic and/or sulfidic form.

### Hydrogenation

SSZ-70 can be used in a catalyst to catalyze Hydrogenation of a hydrocarbon feed containing unsaturated hydrocarbons. The unsaturated hydrocarbons can comprise olefins, dienes, polyeries, aromatic compounds and the like.

Hydrogenation is accomplished by contacting the hydrocarbon feed containing unsaturated hydrocarbons with hydrogen in the presence of a catalyst comprising SSZ-70. The catalyst can also contain one or more metals of Group VIB and Group VIII, including salts, complexes and solutions thereof. Reference to these catalytically active metals is intended to encompass such metals or metals in the elemental state or in some form such as an oxide, sulfide, halide, carboxylate and the like. Examples of such metals include metals, salts or complexes wherein the metal is selected from the group consisting of platinum, palladium, rhodium, iridium or combinations thereof, or the group consisting of nickel, molybdenum, cobalt, tungsten, titanium, chromium, vanadium, rhenium, manganese and combinations thereof.

The hydrogenation component of the catalyst (i.e., the aforementioned metal) is present in an amount effective to provide the hydrogenation function of the catalyst, preferably in the range of from 0.05 to 25% by weight.

Hydrogenation conditions, such as temperature, pressure, space velocities, contact time and the like are well known in the art.

In accordance with this invention, there is provided a process for the reduction of oxides of nitrogen contained in a gas stream wherein said process comprises contacting the gas stream with a molecular sieve, the molecular sieve having a mole ratio greater than about 15 of an oxide of a first tetravalent element to an oxide of a second tetravalent element different from said first tetravalent element, trivalent element, pentavalent element or mixture thereof and having, after calcination, the X-ray diffraction lines of Table II. There is also provided a process for the reduction of oxides of nitrogen contained in a gas stream in the presence of oxygen wherein said process comprises contacting the gas stream with a molecular sieve, the molecular sieve having a mole ratio greater than about 15 of (1) silicon oxide to (2) an oxide selected from aluminum oxide, gallium oxide, iron oxide, boron oxide, titanium oxide, indium oxide and mixtures thereof, and having, after calcination, the X-ray diffraction lines of Table II. The molecular sieve may contain a metal or metal ions (such as cobalt, copper, platinum, iron, chromium, manganese, nickel, zinc, lanthanum, palladium, rhodium or mixtures thereof) capable of catalyzing the reduction of the oxides of nitrogen, and the process may be conducted in the presence of a stoichiometric excess of oxygen. In a preferred embodiment, the gas stream is the exhaust stream of an internal combustion engine.

SSZ-70 may be used for the catalytic reduction of the oxides of nitrogen in a gas stream. Typically, the gas stream also contains oxygen, often a stoichiometric excess thereof, Also, the SSZ-70 may contain a metal or metal ions within or on it which are capable of catalyzing the reduction of the nitrogen oxides. Examples of such metals or metal ions include cobalt, copper, platinum, iron, chromium, manganese, nickel, zinc, lanthanum, palladium, rhodium and mixtures thereof.

One example of such a process for the catalytic reduction of oxides of nitrogen in the presence of a zeolite is disclosed in U.S. Patent No. 4,297,328, issued October 27, 1981 to Ritscher et al. There, the catalytic process is the combustion of carbon monoxide and hydrocarbons and the catalytic reduction of the oxides of nitrogen contained in a gas stream, such as the exhaust gas from an internal combustion engine. The zeolite used is metal ion-exchanged, doped or loaded sufficiently so as to provide an effective amount of catalytic copper metal or copper ions within or on the zeolite. In addition, the process is conducted in an excess of oxidant, e.g., oxygen.

The molecular sieve of the present invention can be used in a catalyst for acylating an aromatic substrate ArHₙ, where n is at least 1, by reacting the aromatic substrate with an acylating agent in the presence of the catalyst. The product of the acylation reaction is ArHₙ₋₁COR where R is an organic radical.

Examples of the aromatic substrate include, but are not limited to, benzene, toluene, anisole and 2-naphthol. Examples of the acylating agent included, but are not limited to, carboxylic acid derivatives, carboxylic acids, acid anhydrides, esters, and acyl halides.

Reaction conditions are known in the art (see, for example, U. S. Patent No. 6,630,606, issued October 7, 2003 to Poliakoffet al., U. S. Patent No. 6,459,000, issued October 1, 2002 to Choudhary et al., and U. S. Patent No: 6,548,722, issued April 15, 2003 to Choudhary et al.). Typically, the acylation reaction is conducted with a weight ratio of the catalyst to the acylating agent of about 0.03 to about 0.5, a mole ratio of aromatic substrate to acylating agent of about 1.0 to about 20, a reaction temperature in the range of about 20°C to about 200°C, a reaction pressure in the range of about I atm to about 5 atm, and a reaction time of about 0.05 hours to about 20 hours.

The partial oxidation of low value hydrocarbons such as alkanes and alkenes into high value products such as alcohols and epoxides is of great commercial interest. These oxidation products are not only valuable as is, but also as intermediates for specialty chemicals including pharmaceuticals and pesticides.

U.S. Patent No. 4,410,501, issued October 18, 1983 to Esposito et al., discloses a titanium-containing analogue of the all-silica ZSM-5 molecular sieve. This material (known as "TS-1") has been found to be useful in catalyzing a wide range of partial oxidation chemistries, for example the production of catechol and hydroquinone from phenol and hydrogen peroxide (H₂O₂) and the manufacture af propylene oxide and cyclohexanone oxime from propylene and cyclohexanone, respectively. In addition, TS-1 can be used to catalyze the reaction af alkanes and aqueous H₂O₂ to form alcohols and ketones. (See Huybrechts, D.R.C. et al., Nature 1990, 345, 240-242 and Tatsumi, T. et al., J.C.S. Chem. Commun. 1990, 476-477.)

TS-1 has many salient features, other than its catalytic abilities, which make it attractive as a commercial catalyst. Most importantly, it is a solid. This allows for easy separation from the reactants and products (typically liquids) by simple, inexpensive filtration. Moreover, this solid has high thermal stability and a very long lifetime. Calcination in air at moderate temperatures (550°C) restores the material to its original catalytic ability. TS-1 performs best at mild temperatures (<100°C) and pressures (1 atm). The oxidant used for reactions catalyzed by TS-1 is aqueous H₂O₂, which is important because aqueous H₂O₂ is relatively inexpensive and its by-product is water. Hence, the choice of oxidant is favorable from both a commercial and environmental point of view.

While a catalyst system based on TS-1 has many useful features, it has one serious drawback. The zeolite structure of TS-1 includes a regular system of pores which are formed by nearly circular rings often silicon atoms (called 10-membered rings, or simply "10 rings") creating pore diameters of approximately 5.5 Å. This small size results in the exclusion of molecules larger than 5.5 Å. Because the catalytically active sites are located within the pores of the zeolite, any exclusion of molecules from the pores results in poor catalytic activity.

SSZ-70 containing titanium oxide (Ti-SSZ-70) is useful as a catalyst in oxidation reactions, particularly in the oxidation of hydrocarbons. Examples of such reactions include, but are not limited to, the epoxidation of olefins, the oxidation of alkanes, and the oxidation of sulfur-containing, nitrogen-containing or phosphorus-containing compounds.

The amount of Ti-SSZ-70 catalyst employed is not critical, but should be sufficient so as to substantially accomplish the desired oxidation reaction in a practicably short period of time (i.e., a catalytically effective amount). The optimum quantity of catalyst will depend upon a number of factors including reaction temperature, the reactivity and concentration of the substrate, hydrogen peroxide concentration, type and concentration of organic solvent, as well as the activity of the catalyst. Typically, however, the amount of catalyst will be from about 0.001 to 10 grams per mole of substrate.

Typically, the Ti-SSZ-70 is thermally treated (calcined) prior to use as a catalyst.

The oxidizing agent employed in the oxidation processes of this invention is a hydrogen peroxide source such as hydrogen peroxide (H₂O₂) or a hydrogen peroxide precursor (i.e., a compound which under the oxidation reaction conditions is capable of generating or liberating hydrogen peroxide).

The amount of hydrogen peroxide relative to the amount of substrate is not critical, but must be sufficient to cause oxidation of at least some of the substrate. Typically, the molar ratio of hydrogen peroxide to substrate is from about 100:1 1 to about 1:100, preferably 10:1 to about 1:10. When the substrate is an olefin containing more than one carbon-carbon double bond, additional hydrogen peroxide may be required. Theoretically, one equivalent of hydrogen peroxide is required to oxidize one equivalent of a mono-unsaturated substrate, but it may be desirable to employ an excess of one reactant to optimize selectivity to the epoxide. In particular, the use of a moderate to large excess (e.g., 50 to 200%) of olefin relative to hydrogen peroxide may be advantageous for certain substrates.

If desired, a solvent may additionally be present during the oxidation reaction in order to dissolve the reactants other than the Ti-SSZ-70, to provide better temperature control, or to favorably influence the oxidation rates and selectivities. The solvent, if present, may comprise from 1 to 99 weight percent of the total oxidation reaction mixture and is preferably selected such that it is a liquid at the oxidation reaction temperature. Organic compounds having boiling points at atmospheric pressure of from about 50°C to about 150°C are generally preferred for use. Excess hydrocarbon may serve as a solvent or diluent. Illustrative examples of other suitable solvents include, but are not limited to, ketones (e.g., acetone, methyl ethyl ketone, acetophenone), ethers (e.g., tetrahydrofuran, butyl ether), nitriles (e.g., acetonitrile), aliphatic and aromatic hydrocarbons, halogenated hydrocarbons, and alcohols (e.g., methanol, ethanol, isopropyl alcohol, t-butyl alcohol, alpha-methyl benzyl alcohol, cyclohexanol). More than one type of solvent may be utilized. Water may also be employed as a solvent or diluent.

The reaction temperature is not critical, but should be sufficient to accomplish substantial conversion of the substrate within a reasonably short period of time. It is generally advantageous to carry out the reaction to achieve as high a hydrogen peroxide conversion as possible, preferably at least about 50%, more preferably at least about 90%, most preferably at least about 95%, consistent with reasonable selectivities. The optimum reaction temperature will be influenced by catalyst activity, substrate reactivity, reactant concentrations, and type of solvent employed, among other factors, but typically will be in a range of from about 0°C to about 150°C (more preferably from about 25°C to about 120°C). Reaction or residence times from about one minute to about 48 hours (more desirably from about ten minutes to about eight hours) will typically be appropriate, depending upon the above-identified variables. Although subatmospheric pressures can be employed, the reaction is preferably performed at atmospheric or at elevated pressure (typically, between one and 100 atmospheres), especially when the boiling point of the substrate is below the oxidation reaction temperature. Generally, it is desirable to pressurize the reaction vessel sufficiently to maintain the reaction components as a liquid phase mixture. Most (over 50%) of the substrate should preferably be present in the liquid phase.

The oxidation process of this invention maybe carried out in a batch, continuous, or semi-continuous manner using any appropriate type of reaction vessel or apparatus such as a fixed bed, transport bed, fluidized bed, stirred slurry, or CSTR reactor. The reactants may be combined all at once or sequentially. For example, the hydrogen peroxide or hydrogen peroxide precursor may be added incrementally to the reaction zone. The hydrogen peroxide could also be generated in situ within the same reactor zone where oxidation is taking place.

Once the oxidation has been carried out to the desired degree of conversion, the oxidized product may be separated and recovered from the reaction mixture using any appropriate technique such as fractional distillation, extractive distillation, liquid-liquid extraction, crystallization, or the like.

### Olefin Epoxidation

One of the oxidation reactions for which Ti-SSZ-70 is useful as a catalyst is the epoxidation of olefins. The olefin substrate epoxidized in the process of this invention may be any organic compound having at least one ethylenically unsaturated functional group (i.e., a carbon-carbon double bond) and may be a cyclic, branched or straight-chain olefin. The olefin may contain aryl groups (e.g., phenyl, naphthyl). Preferably, the olefin is aliphatic in character and contains from 2 to about 20 carbon atoms. The use of light (low-boiling) C₂ to C₁₀ mono-olefins is especially advantageous.

More than one carbon-carbon double bond may be present in the olefin, i.e., dienes, trienes and other polyunsaturated substrates may be used. The double bond may be in a terminal or internal position in the olefin or may alternatively form part of a cyclic structure (as in cyclooctene, for example).

Other examples of suitable substrates include unsaturated fatty acids or fatty acid derivatives such as esters.

The olefin may contain substituents other than hydrocarbon substituents such as halide, carboxylic acid, ether, hydroxy, thiol, nitro, cyano, ketone, acyl, ester, anhydride, amino, and the like.

Exemplary olefins suitable for use in the process of this invention include ethylene, propylene, the butenes (i.e., 1,2-butene, 2,3-butene, isobutylene), butadiene, the pentenes, isoprene, 1-hexene, 3-hexene, 1-heptene, 1-octene, diisobutylene, 1-nonene, 1-tetradecene, pentamyrcene, camphene, 1-undecene, 1-dodecene, 1-tridecene, 1-tetradecene, 1-pentadecene, 1-hexadecene, 1-heptadecene, 1-octadecene, 1-nonadecene, 1-eicosene, the trimers and tetramers of propylene, cyclopentene, cyclohexene, cycloheptene, cyclooctene, cyclooctadiene, dicyclopentadiene, methylenecyclopropane, methylenecyclopentane, methylenecyclohexane, vinyl cyclohexane, vinyl cyclohexene, methallyl ketone, allyl chloride, the dichlorobutenes, allyl alcohol, allyl carbonate, allyl acetate, alkyl acrylates and methacrylates, diallyl maleate, diallyl phthalate, and unsaturated fatty acids, such as oleic acid, linolenic acid, linoleic acid, erucic acid, palmitoleic acid, and ricinoleic acid and their esters (including mono-, di-, and triglyceride esters) and the like.

Olefins which are especially useful for epoxidation are the C₂-C₂₀ olefins having the general structure

R³R⁴C=CR⁵R⁶

wherein R³, R⁴, R⁵ and R⁶ are the same or different and are selected from the group consisting of hydrogen and C₁-C₁₈ alkyl.

Mixtures of olefins may be epoxidized and the resulting mixtures of epoxides either employed in the mixed form or separated into the different component epoxides.

The present invention further provides a process for oxidation of hydrocarbons comprising contacting said hydrocarbon with hydrogen peroxide in the presence of a catalytically effective amount of Ti-SSZ-70 for a time and at a temperature effective to oxidize said hydrocarbon.

### EXAMPLES

The following examples demonstrate but do not limit the present invention.

### Examples 1-6

### Synthesis of Borosilicate SSZ-70 (B-SSZ-70)

B-SSZ-70 is synthesized by preparing the gel compositions, i.e., reaction mixtures, having the compositions, in terms of mole ratios, shown in the table below. The resulting gel is placed in a Parr bomb reactor and heated in an oven at the temperature (°C) indicated in the table while rotating at 43 rpm. Amounts in the table are in millimoles. Products are analyzed by X-ray diffraction (XRD) and found to be B-SSZ-70 or a mixture of B-SSZ-70 and amorphous material.

| Ex. No. | SiO₂ | DIPI | H₂O/SiO₂ | HF | H₃BO₃ | Temp., °C | Seeds | Days | Prod. |
|---|---|---|---|---|---|---|---|---|---|
| 1 | 18 | 9 | 15 | 9 | 1.0 | 150 | No | 95 | AM/ B-SSZ-70 |
| 2 | 18 | 9 | 15 | 9 | 1.0 | 150 | Yes | 98 | AM/ B-SSZ-70 |
| 3 | 18 | 9 | 15 | 9 | 1.0 | 170 | No | 52 | B-SSZ-70 |
| 4 | 18 | 9 | 15 | 9 | 1.0 | 150 | Yes | 80 | B-SSZ-70 |
| 5 | 18 | 9 | 15 | 9 | 3.3 | 170 | No | 52 | B-SSZ-70 |
| 6 | 18 | 9 | 15 | 9 | 5.0 | 170 | No | 61 | B-SSZ-70 |

| | | | | | | | | | |
|---|---|---|---|---|---|---|---|---|---|
| AM = amorphous material | | | | | | | | | |

The X-ray diffraction lines for as-synthesized SSZ-70 are shown in the table below.

**As-Synthesized SSZ-70 XRD**

| 2 Theta ^{(a)} | d-spacing (Angstroms) | Relative Intensity (%) |
|---|---|---|
| 3.32 | 26.6 | 84 |
| 6.70 | 13.2 | 100 |
| 7.26 | 12.2 | 45 |
| 8.78 | 10.1 | 44 |
| 10.04 | 8.81 | 20 |
| 10.88 | 8.13 | 17 |
| 13.00 | 6.81 | 16 |
| 13.34 | 6.64 | 26 |
| 14.60 | 6.07 | 23 |
| 15.36 | 5.77 | 14 |
| 16.66 | 5.32 | 10 |
| 18.54 | 4.79 | 6 |
| 19.30 | 4.60 | 14 |
| 20.02 | 4.44 | 46 |
| 21.86 | 4.07 | 25 |
| 22.54 | 3.94 | 33 |
| 22.88 | 3.89 | 36 |
| 24.38 | 3.65 | 13 |
| 25.28 | 3.52 | 25 |
| 26.36 | 3.38 | 61 |
| 26.88 | 3.32 | 31 |
| 29.56 | 3.02 | 6 |
| 32.00 | 2.80 | 8 |
| 33.61 | 2.67 | 4 |
| 36.94 | 2.43 | 5 |
| 38.40 | 2.34 | 7 |

| | | |
|---|---|---|
| ^{(a)} ± 0.15 | | |

### Example 7

A run is set up as in the table above but the mole ratios are as follows: SiO₂ = 16 mmoles, DIPI = 5 mmoles, H₃BO₃ = 4 mmoles and water = 240 mmoles. No HF component is used. The reaction is run for only seven days at 43 RPM at 170°C. The product is SSZ-70.

### Example 8

### Calcination of SSZ-70

SSZ-70 is calcined to remove the structure directing agent (SDA) as described below. A thin bed of SSZ-70 in a calcination dish is heated in a muffle furnace from room temperature to 120°C at a rate of 1°C/minute and held for 2 hours. Then, the temperature is ramped up to 540°C at a rate of 1°C/minute and held for 5 hours. The temperature is ramped up again at 1°C/minute to 595°C and held there for 5 hours. A 50/50 mixture of air and nitrogen passes through the muffle furnace at a rate of 20 standard cubic feet (0.57 standard cubic meters) per minute during the calcination process. The XRD lines for calcined SSZ-70 are shown in the table below.

| 2 Theta^{(a)} | d-spacing (Angstroms) | Relative Intensity (%) |
|---|---|---|
| 3.93 | 22.5 | 22 |
| 7.31 | 12.1 | 67 |
| 7.75 | 11.4 | 93 |
| 9.25 | 9.6 | 79 |
| 14.56 | 6.08 | 68 |
| 15.61 | 5.68 | 49 |
| 17.34 | 5.11 | 15 |
| 19.60 | 4.53 | 58 |
| 21.81 | 4.07 | 38 |
| 22.24 | 4.00 | 41 |
| 23.11 | 3.85 | 77 |
| 25.30 | 3.52 | 23 |
| 26.30 | 3.39 | 99 |
| 26.81 | 3.33 | 80 |

| | | |
|---|---|---|
| ^{(a)} ± 0.15 | | |

### Example 9

### Replacement of Boron with Aluminum

Calcined SSZ-70 (about 5 grams) is combined with 500 grams of 1 M aqueous Al(NO₃)₃ solution and treated under reflux for 100 hours. The resulting aluminum-containing SSZ-70 product is then washed with 100 ml 0.01N HCl and then with one liter of water, filtered and air dried at room temperature in a vacuum filter.

### Example 10

### Constraint Index

The hydrogen form of calcined SSZ-70 is pelletized at 3 KPSI, crushed and granulated to 20-40 mesh. A 0.6 gram sample of the granulated material is calcined in air at 540°C for 4 hours and cooled in a desiccator to ensure dryness. Then, 0.5 gram is packed into a 3/8 inch stainless steel tube with alundum on both sides of the molecular sieve bed. A Lindburg furnace is used to heat the reactor tube. Helium is introduced into the reactor tube at 10 cc/min. and at atmospheric pressure. The reactor is heated to about 427°C (800°F), and a 50/50 feed of n-hexane and 3-methylpentane is introduced into the reactor at a rate of 8 µl/min. The feed is delivered by a Brownlee pump. Direct sampling into a GC begins after 10 minutes of feed introduction. The Constraint Index (CI) value is calculated from the GC data using methods known in the art. The results are shown in the table below.

| | | | | |
|---|---|---|---|---|
| Time, Min. | 10 | 40 | 70 | 100 |
| Feed Conv. % | 6.4 | 6.5 | 6.5 | 6.4 |
| CI (excl. 2-MP) | 0.6 | 0.59 | 0.56 | 0.56 |
| CI (incl. 2-MP) | 0.78 | 0.79 | 0.75 | 0.76 |

| | | | | |
|---|---|---|---|---|
| 2-MP = 2-methylpentane | | | | |

### Example 11

### Hydrocracking of n-Hexadecane

A1 gm sample of calcined SSZ-70 is suspended in 10 gm de-ionized water. To this suspension, a solution of Pt(NH₃)₄.(NO₃)₂ at a concentration which would provide 0.5 wt. % Pt with respect to the dry weight of the molecular sieve sample is added. The pH of the solution is adjusted to pH of ∼9 by a drop-wise addition of dilute ammonium hydroxide solution. The mixture is then allowed to stand at 25°C for 48 hours. The mixture is then filtered through a glass frit, washed with de-ionized water, and air-dried. The collected Pt-SSZ-70 sample is slowly calcined up to 288°C in air and held there for three hours.

The calcined Pt/SSZ-70 catalyst is pelletized in a Carver Press and granulated to yield particles with a 20/40 mesh size. Sized catalyst (0.5 g) is packed into a ¼ inch OD tubing reactor in a micro unit for n-hexadecane hydroconversion. The table below gives the run conditions and the products data for the hydrocracking test on n-hexadecane.

The results shown in the table below show that SSZ-70 is effective as a hydrocracking catalyst. The data show that the catalyst has a very high selectivity for hydrocracking to linear paraffins, rather than isomerization selectivity. Also, a high ratio of liquid/gas (C₅₊/C₄₋) is achieved.

| | | |
|---|---|---|
| Temperature | 660°F (349°C) | 690°F (366°C) |
| Time-on-Stream (hrs.) | 40 hours | 53 hours |
| PSIG | 2200 | 2200 |
| Titrated? | No | No |
| n-16, % Conversion | 52% | 89% |
| Isomerization Selectivity, % | 5.1 | 2.2 |
| C₅₊/C₄₋ | 11.5 | 7.0 |
| C₄-C₁₃ i/n | 0.02 | 0.03 |

### Example 12

### Micropore Volume

SSZ-70 has a micropore volume of 0.071 cc/gm based on argon adsorption isotherm at 87.5° K (-186°C) recorded on ASAP 2010 equipment from Micromerities. The sample is first degassed at 400°C for 16 hours prior to argon adsorption. The low-pressure dose is 2.00 cm³/g (STP). A maximum of one hour equilibration time per dose is used and the total run time is 37 hours. The argon adsorption isotherm is analyzed using the density function theory (DFT) formalism and parameters developed for activated carbon slits by Olivier (*Porous Mater.* 1995, 2, 9) using the Saito Foley adaptation of the Horvarth-Kawazoe formalism (*Microporous Materials,* 1995, 3, 531) and the conventional t-plot method (J. Catalysis, 1965, 4, 319) (micropore volume by the t-plot method is 0.074 cc/gm).

## Claims

1. A molecular sieve having a mole ratio greater than 15 of (1) an oxide of a first tetravalent element to (2) an oxide of a trivalent element, pentavalent element, second tetravalent element which is different from said first tetravalent element or mixture thereof and having, after calcination, the X-ray diffraction lines of Table II.
**Table II**
| *2 Theta* | *d-spacing (Angstroms)* | *Relative Intensity (%)* |
|---|---|---|
| 7.31 ± 0.15 | 12.1 | VS |
| 7.75 ± 0.15 | 11.4 | VS |
| 9.25 ± 0.15 | 9.6 | VS |
| 14.56 ± 0.15 | 6.08 | VS |
| 15.61 ± 0.15 | 5.68 | S |
| 19.60 ± 0.15 | 4.53 | S |
| 21.81 ± 0.15 | 4.07 | M |
| 22.24 ± 0.15 | 4.00 | M-S |
| 26.30 ± 0.15 | 3.39 | VS |
| 26.81 ± 0.15 | 3.33 | VS |

2. The molecular sieve of claim 1, wherein the molecular sieve has a mole ratio greater than 15 of (1) silicon oxide to (2) an oxide selected from aluminum oxide, gallium oxide, iron oxide, boron oxide, titanium oxide, indium oxide and mixtures thereof.

3. A molecular sieve according to claim 2 wherein the oxides comprise silicon oxide and aluminum oxide.

4. A molecular sieve according to claim 2 wherein the oxides comprise silicon oxide and boron oxide.

5. A molecular sieve according to claim 2 wherein the oxides comprise silicon oxide and titanium oxide.

6. A molecular sieve according to claim 2 wherein the molecular sieve comprises essentially all silicon oxide.

7. A molecular sieve according to claim 1 wherein said molecular sieve is predominantly in the hydrogen form, wherein "predominantly in the hydrogen form" means that after calcination at least 80% of the cation sites in said molecular sieve are occupied by hydrogen ions and/or rare earth ions.

8. A molecular sieve according to claim 1 wherein said molecular sieve is substantially free of acidity.

9. A molecular sieve according to claim 2 wherein said molecular sieve is predominantly in the hydrogen form, wherein "predominantly in the hydrogen form" means that after calcination at least 80% of the cation sites in said molecular sieve are occupied by hydrogen ions and/or rare earth ions.

10. A molecular sieve according to claim 2 wherein said molecular sieve is substantially free of acidity.

11. The molecular sieve of claim 1 having a composition, as synthesized and in the anhydrous state, in terms of mole ratios as follows:
| | |
|---|---|
| YO₂/B₂O₃ | 20 - 60 |
| M_{2/n}/YO₂ | 0.01 - 0.03 |
| Q/YO₂ | 0.02 - 0.05 |
| F/YO₂ | 0 - 0.10 |
wherein Y is silicon; M is an alkali metal cation, alkaline earth metal cation or mixtures thereof; n is the valence of M; F is fluorine and Q is a N, N'-diisopropyl imidazolium cation.

12. A method of preparing the molecular sieve of claim 4, said method comprising contacting under crystallization conditions sources of said oxides, a structure directing agent comprising a N, N'-diisopropyl imidazolium cation and fluoride ions.

13. The method according to claim 12 wherein the molecular sieve is prepared from a reaction mixture comprising, in term of mole ratios:
| | |
|---|---|
| YO₂/B₂O₃ | 5 - 60 |
| OH-/YO₂ | 0.10 - 0.50 |
| Q/YO₂ | 0.05 - 0.50 |
| M_{2/n}/YO₂ | 0.02 - 0.40 |
| H₂O/YO₂ | 30 - 80 |
| F/YO₂ | 0 - 0.50 |
where Y is silicon; M is an alkali metal cation, alkaline earth metal cation or mixtures thereof; n is the valence of M; F is fluorine and Q is a N, N'-diisopropyl imidazolium cation.

14. A process for converting hydrocarbons comprising contacting a hydrocarbonaceous feed at hydrocarbon converting conditions with a catalyst comprising the molecular sieve of claim 1 or claim 2.

15. The process of claim 14 wherein the molecular sieve is substantially free of acidity.

16. The process of claim 14 wherein the process is a hydrocracking process comprising contacting the catalyst with a hydrocarbon feedstock under hydrocracking conditions.

17. The process of claim 14 wherein the process is a dewaxing process comprising contacting the catalyst with a hydrocarbon feedstock under dewaxing conditions.

18. The process of claim 14 wherein the process is a process for improving the viscosity index of a dewaxed product of waxy hydrocarbon feeds comprising contacting the catalyst with a waxy hydrocarbon feed under isomerization dewaxing conditions.

19. The process of claim 14 wherein the process is a process for producing a C₂₀₊ lube oil from a C₂₀₊ olefin feed comprising isomerizing said olefin feed under isomerization conditions over the catalyst.

20. The process of claim 19 wherein the catalyst further comprises at least one Group VIII metal.

21. The process of claim 14 wherein the process is a process for catalytically dewaxing a hydrocarbon oil feedstock boiling above 350°F (177°C) and containing straight chain and slightly branched chain hydrocarbons comprising contacting said hydrocarbon oil feedstock in the presence of added hydrogen gas at a hydrogen pressure of 15-3000 psi (0.103-20.7 MPa) under dewaxing conditions with the catalyst.

22. The process of claim 21 wherein the catalyst further comprises at least one Group VIII metal.

23. The process of claim 21 wherein said catalyst comprises a layered catalyst comprising a first layer comprising the molecular sieve and at least one Group VIII metal, and a second layer comprising an aluminosilicate zeolite which is more shape selective than the molecular sieve of said first layer.

24. The process of claim 14 wherein the process is a process for preparing a lubricating oil which comprises:
hydrocracking in a hydrocracking zone a hydrocarbonaceous feedstock to obtain an effluent comprising a hydrocracked oil; and
catalytically dewaxing said effluent comprising hydrocracked oil at a temperature of at least 400°F (204°C) and at a pressure of from 15 psig to 3000 psig (0.103 to 20.7 MPa gauge) in the presence of added hydrogen gas with the catalyst.

25. The process of claim 24 wherein the catalyst further comprises at least one Group VIII metal.

26. The process of claim 14 wherein the process is a process for isomerization dewaxing a raffinate comprising contacting said raffinate in the presence of added hydrogen under isomerization dewaxing conditions with the catalyst.

27. The process of claim 26 wherein the catalyst further comprises at least one Group VIII metal.

28. The process of claim 26 wherein the raffinate is bright stock.

29. The process of claim 14 wherein the process is a process for increasing the octane of a hydrocarbon feedstock to produce a product having an increased aromatics content comprising contacting a hydrocarbonaceous feedstock which comprises normal and slightly branched hydrocarbons having a boiling range above 40°C and less than 200°C under aromatic conversion conditions with the catalyst.

30. The process of claim 29 wherein the molecular sieve is substantially free of acid.

31. The process of claim 29 wherein the catalyst further comprises a Group VIII metal component.

32. The process of claim 14 wherein the process is a catalytic cracking process comprising contacting a hydrocarbon feedstock in a reaction zone under catalytic cracking conditions in the absence of added hydrogen with the catalyst.

33. The process of claim 32 wherein the catalyst additionally comprises a large pore crystalline cracking component.

34. The process of claim 14 wherein the process is an isomerization process for isomerizing C₄ to C₇ hydrocarbons, comprising contacting a feed having normal and slightly branched C₄ to C₇ hydrocarbons under isomerizing conditions with the catalyst.

35. The process of claim 34 wherein the molecular sieve has been impregnated with at least one Group VIII metal.

36. The process of claim 35 wherein the catalyst has been calcined in a steam/air mixture at an elevated temperature after impregnation of the Group VIII metal.

37. The process of claim 35 wherein the Group VIII metal is platinum.

38. The process of claim 14 wherein the process is a process for alkylating an aromatic hydrocarbon which comprises contacting under alkylation conditions at least a molar excess of an aromatic hydrocarbon with a C₂ to C₂₀ olefin under at least partial liquid phase conditions and in the presence of the catalyst.

39. The process of claim 38 wherein the olefin is a C₂ to C₄ olefin.

40. The process of claim 39 wherein the aromatic hydrocarbon and olefin are present in a molar ratio of 4:1 to 20:1, respectively.

41. The process of claim 39 wherein the aromatic hydrocarbon is selected from the group consisting of benzene, toluene, ethylbenzene, xylene, naphthalene, naphthalene derivatives, dimethylnaphthalene or mixtures thereof.

42. The process of claim 14 wherein the process is a process for transalkylating an aromatic hydrocarbon which comprises contacting under transalkylating conditions an aromatic hydrocarbon with a polyalkyl aromatic hydrocarbon under at least partial liquid phase conditions and in the presence of the catalyst.

43. The process of claim 42 wherein the aromatic hydrocarbon and the polyalkyl aromatic hydrocarbon are present in a molar ratio of from 1:1 to 25:1, respectively.

44. The process of claim 42 wherein the aromatic hydrocarbon is selected from the group consisting of benzene, toluene, ethylbenzene, xylene, or mixtures thereof.

45. The process of claim 42 wherein the polyalkyl aromatic hydrocarbon is a dialkylbenzene.

46. The process of claim 14 wherein the process is a process to convert paraffins to aromatics which comprises contacting paraffins under conditions which cause paraffins to convert to aromatics with a catalyst comprising the molecular sieve and gallium, zinc, or a compound of gallium or zinc.

47. The process of claim 14 wherein the process is a process for isomerizing olefins comprising contacting said olefin under conditions which cause isomerization of the olefin with the catalyst.

48. The process of claim 14 wherein the process is a process for isomerizing an isomerization feed comprising an aromatic C₈ stream of xylene isomers or mixtures of xylene isomers and ethylbenzene, wherein a more nearly equilibrium ratio of ortho-, meta and para-xylenes is obtained, said process comprising contacting said feed under isomerization conditions with the catalyst.

49. The process of claim 14 wherein the process is a process for oligomerizing olefins comprising contacting an olefin feed under oligomerization conditions with the catalyst.

50. The process of claim 14, wherein the process is a process for converting oxygenated hydrocarbons comprising contacting said oxygenated hydrocarbon under conditions to produce liquid products with the catalyst.

51. The process of claim 50 wherein the oxygenated hydrocarbon is a lower alcohol having 1 to 10 carbon atoms.

52. The process of claim 51 wherein the lower alcohol is methanol.

53. The process of claim 14 wherein the process is a process for the production of higher molecular weight hydrocarbons from lower molecular weight hydrocarbons comprising the steps of:
(a) introducing into a reaction zone a lower molecular weight hydrocarbon-containing gas and contacting said gas in said zone under C₂₊ hydrocarbon synthesis conditions with the catalyst and a metal or metal compound capable of converting the lower molecular weight hydrocarbon to a higher molecular weight hydrocarbon; and
(b) withdrawing from said reaction zone a higher molecular weight hydrocarbon-containing stream.

54. The process of claim 53 wherein the metal or metal compound comprises a lanthanide or actinide metal or metal compound.

55. The process of claim 53 wherein the lower molecular weight hydrocarbon is methane.

56. A catalyst composition for promoting polymerization of 1-olefins, said composition comprising
(A) the molecular sieve of claim 1; and
(B) an organotitanium or organochromium compound.

57. The catalyst composition of claim 56 wherein oxide (1) is silicon oxide, and oxide (2) is an oxide selected from aluminum oxide, gallium oxide, iron oxide, boron oxide, titanium oxide, indium oxide.

58. The process of claim 14 wherein the process is a process for polymerizing 1-olefins, which process comprises contacting 1-olefin monomer with a catalytically effective amount of the catalyst composition of claim 56 under polymerization conditions which include a temperature and pressure suitable for initiating and promoting the polymerization reaction.

59. The process of claim 58 wherein oxide (1) is silicon oxide, and oxide (2) is an oxide selected from aluminum oxide, gallium oxide, iron oxide, boron oxide, titanium oxide, indium oxide.

60. The process of claim 58 wherein the 1-olefin monomer is ethylene.

61. The process of claim 59 wherein the 1-olefin monomer is ethylene.

62. The process of claim 14 wherein the process is a process for hydrogenating a hydrocarbon feed containing unsaturated hydrocarbons, the process comprising contacting the feed with hydrogen under conditions which cause hydrogenation with the catalyst.

63. The process of claim 62 wherein the catalyst contains metals, salts or complexes wherein the metal is selected from the group consisting of platinum, palladium, rhodium, iridium or combinations thereof, or the group consisting of nickel, molybdenum, cobalt, tungsten, titanium, chromium, vanadium, rhenium, manganese and combinations thereof.

64. A process for hydrotreating a hydrocarbon feedstock comprising contacting the feedstock with a hydrotreating catalyst and hydrogen under hydrotreating conditions, wherein the catalyst comprises the molecular sieve of claim 1.

65. The process of claim 64 wherein the catalyst contains a Group VIII metal or compound, a Group VI metal or compound or combinations thereof.

66. The process of claim 64 wherein oxide (1) is silicon oxide, and oxide (2) is an oxide selected from aluminum oxide, gallium oxide, iron oxide, boron oxide, titanium oxide, indium oxide.

67. The process of claim 14, wherein the catalyst comprises the molecular sieve of claim 2.

68. The process of claim 67 wherein the molecular sieve is substantially free of acidity.

69. The process of claim 14, 16, 17, 18, 19, 21, 24, 26, 32, 34, 38, 42 or 67 wherein the molecular sieve is predominantly in the hydrogen form, wherein "predominantly in the hydrogen form" means that after calcination at least 80% of the cation sites in the molecular sieve are occupied by hydrogen ions and/or rare earth ions.

70. A process for the reduction of oxides of nitrogen contained in a gas stream wherein said process comprises contacting the gas stream with the molecular sieve of claim 1.

71. A process for the reduction of oxides of nitrogen contained in a gas stream in the presence of oxygen wherein said process comprises contacting the gas stream with the molecular sieve of claim 2.

72. The process of claim 70 or 71 conducted in the presence of oxygen.

73. The process of claim 70 or 71 wherein said molecular sieve contains a metal or metal ions capable of catalyzing the reduction of the oxides of nitrogen.

74. The process of claim 73 wherein the metal is cobalt, copper, platinum, iron, chromium, manganese, nickel, zinc, lanthanum, palladium, rhodium or mixtures thereof

75. The process of claim 70 or 71 wherein the gas stream is the exhaust stream of an internal combustion engine.

76. The process of claim 74 wherein the gas stream is the exhaust stream of an internal combustion engine.

77. A process for oxidation of hydrocarbons comprising contacting said hydrocarbon with an oxidizing agent in the presence of a catalytically effective amount of the molecular sieve of claim 5.

78. The process of claim 77, wherein the process is a process for epoxidation of an olefin comprising contacting said olefin with hydrogen peroxide in the presence of the molecular sieve for a time and at a temperature effective to epoxidize said olefin.

79. The process of claim 77, wherein the process is a process for oxidizing cyclohexane comprising contacting said cyclohexane with hydrogen peroxide in the presence of a catalytically effective amount of the molecular sieve for a time and at a temperature effective to oxidize said cyclohexane.

80. A catalytic oxidation process comprising contacting under oxidation conditions (1) a reactant which is catalytically oxidizable in the presence of hydrogen peroxide, (2) aqueous hydrogen peroxide and (3) a catalytically effective amount of an oxidation catalyst comprising the molecular sieve of claim 5.

81. The process of claim 80 wherein the elemental mole ratio of titanium to silicon is 0.005 to 0.2.

82. The process of claim 80 wherein the elemental mole ratio of titanium to silicon is 0.01 to 0.05.

83. The process of claim 80 wherein the oxidizable reactant is a hydrocarbon.

## Patentansprüche

1. Molekularsieb mit einem Molarverhältnis größer als 15 zwischen (1) einem Oxid eines ersten vierwertigen Elements und (2) einem Oxid eines dreiwertigen Elements, eines fünfwertigen Elements, eines zweiten vierwertigen Elements, das sich vom ersten vierwertigen Element unterscheidet, oder einem Gemisch davon und mit, nach Kalzinierung, den Röntgendiffraktionslinien aus Tafel II.
| *2-Theta* | *d-Spacing (angström)* | *Relative Intensität* (%) |
|---|---|---|
| 7,31 ± 0,15 | 12,1 | VS |
| 7,75 ± 0,15 | 11,4 | VS |
| 9,25 ± 0,15 | 9,6 | VS |
| 14,56 ± 0,15 | 6,08 | VS |
| 15,61 ± 0,15 | 5,68 | S |
| 19,60 ± 0,15 | 4,53 | S |
| 21,81 ±0,15 | 4,07 | M |
| 22,24 ± 0,15 | 4,00 | M-S |
| 26,30 ± 0,15 | 3,39 | VS |
| 26,81 ± 0,15 | 3,33 | VS |

2. Molekularsieb gemäß Anspruch 1, wobei das Molekularsieb ein Molarverhältnis größer als 15 hat zwischen (1) Siliciumoxid und (2) einem Oxid, ausgewählt aus Aluminiumoxid, Galliumoxid, Eisenoxid, Boroxid, Titanoxid, Indiumoxid und Gemischen davon.

3. Molekularsieb gemäß Anspruch 2, wobei die Oxide Siliciumoxid und Aluminiumoxid umfassen.

4. Molekularsieb gemäß Anspruch 2, wobei die Oxide Siliciumoxid und Boroxid umfassen.

5. Molekularsieb gemäß Anspruch 2, wobei die Oxide Siliciumoxid und Titanoxid umfassen.

6. Molekularsieb gemäß Anspruch 2, wobei das Molekularsieb im Wesentlichen alles Siliciumoxid umfasst.

7. Molekularsieb gemäß Anspruch 1, wobei das Molekularsieb vorwiegend in der Wasserstoffform ist, wobei "vorwiegend in der Wasserstoffform" bedeutet, dass nach Kalzinierung mindestens 80% der Kationenorte im Molekularsieb durch Wasserstoffionen und/oder Ionen seltener Erden besetzt sind.

8. Molekularsieb gemäß Anspruch 1, wobei das Molekularsieb im Wesentlichen frei von Säure ist.

9. Molekularsieb gemäß Anspruch 2, wobei das Molekularsieb vorwiegend in der Wasserstoffform ist, wobei "vorwiegend in der Wasserstoffform" bedeutet, dass nach Kalzinierung mindestens 80% der Kationenorte im Molekularsieb durch Wasserstoffionen und/oder Ionen seltener Erden besetzt sind.

10. Molekularsieb gemäß Anspruch 2, wobei das Molekularsieb im Wesentlichen frei von Säure ist.

11. Molekularsieb gemäß Anspruch 1, das eine Zusammensetzung, wie synthetisiert und im anhydrischen Zustand, in folgenden Molarverhältnissen hat:
| | |
|---|---|
| YO₂/B₂O₃ | 20 bis 60 |
| M_{2/n}/YO₂ | 0,01 bis 0,03 |
| Q/YO₂ | 0,02 bis 0,05 |
| F/YO₂ | 0 bis 0,10 |
worin Y Silicium ist; M ein Alkalimetallkation, Alkalierdmetallkation oder ein Gemisch davon ist; n die Wertigkeit von M ist; F Fluor ist und Q ein N,N'-Diisopropylimidazoliumkation ist.

12. Herstellungsverfahren für das Molekularsieb aus Anspruch 4, das Verfahren umfassend Zusammenbringen und Kristallisationsbedingungen von Quellen der Oxide, ein Strukturführungsmittel, umfassend ein N,N`-Diisopropylimidazoliumkation und Fluoridionen.

13. Verfahren gemäß Anspruch 12, wobei das Molekularsieb hergestellt wird aus einem Reaktionsgemisch, umfassend in Molarverhältnissen:
| | |
|---|---|
| YO₂/B₂O₃ | 5 bis 60 |
| OH-/YO₂ | 0,10 bis 0,50 |
| Q/YO₂ | 0,05 bis 0,50 |
| M_{2/n}/YO₂ | 0,02 bis 0,40 |
| H₂O/YO₂ | 30 bis 80 |
| F/YO₂ | 0 bis 0,50 |
worin Y Silicium ist; M ein Alkalimetallkation, Alkalierdmetallkation oder ein Gemisch davon ist; n die Wertigkeit von M ist; F Fluor ist und Q ein N,N'-Diisopropylimidazoliumkation ist.

14. Verfahren zum Umwandeln von Kohlenwasserstoffen, umfassend Zusammenbringen einer Kohlenwasserstoffzufuhr bei Kohlenwasserstoff-Umwandlungsbedingungen mit einem Katalysator, umfassend das Molekularsieb aus Anspruch 1 oder Anspruch 2.

15. Verfahren gemäß Anspruch 14, wobei das Molekularsieb im Wesentlichen frei von Säure ist.

16. Verfahren gemäß Anspruch 14, wobei das Verfahren ein Hydrocracking-Verfahren ist, umfassend Zusammenbringen des Katalysators mit einer Kohlenwasserstoffzufuhr unter Hydrocracking-Bedingungen.

17. Verfahren gemäß Anspruch 14, wobei das Verfahren ein Entwachsungsverfahren ist, umfassend Zusammenbringen des Katalysators mit einer Kohlenwasserstoffzufuhr unter Entwachsungsbedingungen.

18. Verfahren gemäß Anspruch 14, wobei das Verfahren ein Verfahren ist zum Verbessern des Viskositätsindex eines entwachsten Produkts aus wachsigen Kohlenwasserstoffzufuhren, umfassend Zusammenbringen des Katalysators mit einer wachsigen Kohlenwasserstoffzufuhr unter Isomerisierungs-Entwachsungsbedingungen.

19. Verfahren gemäß Anspruch 14, wobei das Verfahren ein Verfahren ist zum Herstellen eines C₂₀₊-Schmieröls aus einer C₂₀₊-Olefinzufuhr, umfassend Isomerisieren der Olefinzufuhr unter Isomerisierungsbedingungen über dem Katalysator.

20. Verfahren gemäß Anspruch 19, wobei der Katalysator mindestens ein Metall der Gruppe VIII umfasst.

21. Verfahren gemäß Anspruch 14, wobei das Verfahren ein Verfahren ist zum katalytischen Entwachsen einer Kohlenwasserstofföl-Zufuhr, die über 350°F (177°C) siedet und lineare und leicht verzweigte Kohlenwasserstoffe enthält, umfassend Zusammenbringen der Kohlenwasserstofföl-Zufuhr in der Anwesenheit von zugefügtem Wasserstoffgas bei einem Wasserstoffdruck von 15 bis 3000 psi (0,103 bis 20,7 MPa) unter Entwachsungsbedingungen mit dem Katalysator.

22. Verfahren gemäß Anspruch 21, wobei der Katalysator mindestens ein Metall der Gruppe VIII umfasst.

23. Verfahren gemäß Anspruch 21, wobei der Katalysator einen geschichteten Katalysator umfasst, umfassend eine erste Schicht, umfassend das Molekularsieb und mindestens ein Metall der Gruppe VIII, und eine zweite Schicht, umfassend ein Aluminosilicat-Zeolit, das stärker formselektiv ist, als das Molekularsieb der ersten Schicht.

24. Verfahren gemäß Anspruch 14, wobei das Verfahren ein Herstellungsverfahren für ein Schmieröl ist, das umfasst
Hydrocracken in einem Hydrocracking-Bereich einer Kohlenwasserstoffzufuhr zum Erhalten eines Ausflusses, umfassend ein hydrogecracktes Öl; und katalytisches Entwachsen des Ausflusses, umfassend hydrogecracktes Öl, bei einer Temperatur von mindestens 400°F (204°C) und einem Druck von 15 psig bis 3000 psig (0,103 bis 20,7 MPa Überdruck) in der Anwesenheit von zugefügtem Wasserstoffgas mit dem Katalysator.

25. Verfahren gemäß Anspruch 24, wobei der Katalysator mindestens ein Metall der Gruppe VIII umfasst.

26. Verfahren gemäß Anspruch 14, wobei das Verfahren ein Verfahren ist zur Isomerisierungsentwachsung eines Raffinats, umfassend Zusammenbringen des Raffinats in der Anwesenheit von zugefügtem Wasserstoff unter Isomerisierungs-Entwachsungsbedingungen mit dem Katalysator.

27. Verfahren gemäß Anspruch 26, wobei der Katalysator mindestens ein Metall der Gruppe VIII umfasst.

28. Verfahren gemäß Anspruch 26, wobei das Raffinat Brightstock ist.

29. Verfahren gemäß Anspruch 14, wobei das Verfahren ein Verfahren ist zum Erhöhen des Oktanwerts einer Kohlenwasserstoffzufuhr zum Herstellen eines Produkts mit einem erhöhtem Aromatgehalt, umfassend Zusammenbringen einer Kohlenwasserstoffzufuhr, die normale und leicht verzweigte Kohlenwasserstoffe umfasst mit einem Siedebereich über 40°C und unter 200°C, unter aromatischen Umwandlungsbedingungen mit dem Katalysator.

30. Verfahren gemäß Anspruch 29, wobei das Molekularsieb im Wesentlichen frei von Säure ist.

31. Verfahren gemäß Anspruch 29, wobei der Katalysator mindestens ein Metall der Gruppe VIII umfasst.

32. Verfahren gemäß Anspruch 14, wobei das Verfahren ein katalytisches Cracking-Verfahren ist, umfassend Zusammenbringen einer Kohlenwasserstoffzufuhr in einer Reaktionszone unter katalytischen Cracking-Bedingungen in der Abwesenheit von zugefügtem Wasserstoff mit dem Katalysator.

33. Verfahren gemäß Anspruch 32, wobei der Katalysator zusätzlich einen großporigen kristallinen Cracking-Bestandteil umfasst.

34. Verfahren gemäß Anspruch 14, wobei das Verfahren ein Isomerisierungsverfahren ist für die Isomerisierung von C₄- bis C₇-Kohlenwasserstoffen, umfassend Zusammenbringen einer Zufuhr mit normalen und leicht verzweigten C₄- bis C₇-Kohlenwasserstoffen unter Isomerisierungsbedingungen mit dem Katalysator.

35. Verfahren gemäß Anspruch 34, wobei das Molekularsieb mit mindestens einem Metall der Gruppe VIII imprägniert worden ist.

36. Verfahren gemäß Anspruch 35, wobei der Katalysator in einem Dampf-Wassergemisch bei erhöhter Temperatur nach Imprägnierung mit dem Metall der Gruppe VIII kalziniert worden ist.

37. Verfahren gemäß Anspruch 35, wobei das Metall der Gruppe VIII Platin ist.

38. Verfahren gemäß Anspruch 14, wobei das Verfahren ein Verfahren ist zum Alkylieren eines aromatischen Kohlenwasserstoffs, umfassend Zusammenbringen unter Alkylierungsbedingungen mindestens eines molaren Überschusses eines aromatischen Kohlenwasserstoffs mit einem C₂- bis C₂₀-Olefin unter mindestens teilweise Flüssigphasenbedingungen und in der Anwesenheit des Katalysators.

39. Verfahren gemäß Anspruch 38, wobei das Olefin ein C₂- bis C₄-Olefin ist.

40. Verfahren gemäß Anspruch 39, wobei der aromatische Kohlenwasserstoff und das Olefin jeweils in einem Molarverhältnis von 4:1 bis 20:1 anwesend sind.

41. Verfahren gemäß Anspruch 39, wobei der aromatische Kohlenwasserstoff ausgewählt ist aus der Gruppe Benzol, Toluol, Ethylbenzol, Xylol, Naphthalin, Naphthalin-Derivate, Dimethylnaphthalin oder Gemische davon.

42. Verfahren gemäß Anspruch 14, wobei das Verfahren ein Verfahren ist zum Transalkylieren eines aromatischen Kohlenwasserstoffs, umfassend Zusammenbringen unter Transalkylierungsbedingungen eines aromatischen Kohlenwasserstoffs mit einem aromatischen Polyalkylkohlenwasserstoff unter mindestens teilweise Flüssigphasenbedingungen und in der Anwesenheit des Katalysators.

43. Verfahren gemäß Anspruch 42, wobei der aromatische Kohlenwasserstoff und der aromatische Polyalkylkohlenwasserstoff jeweils in einem Molarverhältnis von 1:1 bis 25:1 anwesend sind.

44. Verfahren gemäß Anspruch 42, wobei der aromatische Kohlenwasserstoff ausgewählt ist aus der Gruppe Benzol, Toluol, Ethylbenzol, Xylol oder Gemische davon.

45. Verfahren gemäß Anspruch 42, wobei der aromatische Polyalkylkohlenwasserstoff ein Dialkylbenzol ist.

46. Verfahren gemäß Anspruch 14, wobei das Verfahren ein Verfahren ist zum Umwandeln von Paraffinen in Aromate, umfassend Zusammenbringen von Paraffinen unter Bedingungen, die auslösen, dass Paraffine sich in Aromate umwandeln, mit einem Katalysator, umfassend das Molekularsieb und Gallium, Zink oder eine Verbindung aus Gallium oder Zink.

47. Verfahren gemäß Anspruch 14, wobei das Verfahren ein Verfahren ist zum Isomerisieren von Olefinen, umfassend Zusammenbringen des Olefins unter Bedingungen, die Isomerisierung des Olefins auslösen, mit dem Katalysator.

48. Verfahren gemäß Anspruch 14, wobei das Verfahren ein Verfahren ist zum Isomerisieren einer Isomerisierungszufuhr, umfassend eine aromatische C₈-Zufuhr aus Xylol-Isomeren oder Gemische aus Xylol-Isomeren und Ethylbenzol, wobei ein besseres Gleichgewichtsverhältnis von ortho-, meta- und para-Xylolen erhalten wird, das Verfahren umfassend Zusammenbringen der Zufuhr unter Isomerisierungsbedingungen mit dem Katalysator.

49. Verfahren gemäß Anspruch 14, wobei das Verfahren ein Verfahren ist zum Oligomerisieren von Olefinen, umfassend Zusammenbringen einer Olefinzufuhr unter Oligomerisierungsbedingungen mit dem Katalysator.

50. Verfahren gemäß Anspruch 14, wobei das Verfahren ein Verfahren ist zum Umwandeln von oxygenierten Kohlenwasserstoffen, umfassend Zusammenbringen der oxygenierten Kohlenwasserstoffe unter Bedingungen zum Herstellen von flüssigen Produkten, mit dem Katalysator.

51. Verfahren gemäß Anspruch 50, wobei der oxygenierte Kohlenwasserstoff ein niederer Alkohol mit 1 bis 10 Kohlenstoffatomen ist.

52. Verfahren gemäß Anspruch 51, wobei der niedere Alkohol Methanol ist.

53. Verfahren gemäß Anspruch 14, wobei das Verfahren ein Herstellungsverfahren ist für Kohlenwasserstoffe mit höherem Molekulargewicht aus Kohlenwasserstoffen mit niedrigerem Molekulargewicht, umfassend die Schritte
(a) Einführen eines Gases, enthaltend Kohlenwasserstoffe mit niedrigerem Molekulargewicht, in eine Reaktionszone und Zusammenbringen des Gases in der Zone unter C₂₊-Kohlenwasserstoff-Synthesebedingungen mit dem Katalysator und einem Metall oder einer Metallverbindung, fähig, einen Kohlenwasserstoff mit niedrigerem Molekulargewicht in einen Kohlenwasserstoff mit höherem Molekulargewicht umzuwandeln; und
(b) Entfernen aus der Reaktionszone eines Stroms, enthaltend Kohlenwasserstoff mit höherem Molekulargewicht.

54. Verfahren gemäß Anspruch 53, wobei das Metall oder die Metallverbindung ein Lanthaniden- oderActinidenmetall oder eine solche Metallverbindung umfasst.

55. Verfahren gemäß Anspruch 53, wobei der Kohlenwasserstoff mit niedrigerem Molekulargewicht Methan ist.

56. Katalysatorzusammensetzung zum Fördern von Polymerisierung von 1-Olefinen, die Zusammensetzung umfassend
(A) das Molekularsieb aus Anspruch 1; und
(B) eine Organotitan- oder Organochromverbindung.

57. Katalysatorzusammensetzung gemäß Anspruch 56, wobei Oxid (1) Siliciumoxid ist und Oxid (2) ein Oxid, ausgewählt aus Aluminiumoxid, Galliumoxid, Eisenoxid, Boroxid, Titanoxid, Indiumoxid.

58. Verfahren gemäß Anspruch 14, wobei das Verfahren ein Verfahren ist zum Polymerisieren von 1-Olefinen, das Verfahren umfassend Zusammenbringen von 1-Olefinmonomer mit einer katalytisch wirksamen Menge der Katalysatorzusammensetzung aus Anspruch 56 unter Polymerisierungsbedingungen, die eine Temperatur und einen Druck aufweisen, geeignet für das Einleiten und Fördern der Polymerisierungsreaktion.

59. Verfahren gemäß Anspruch 58, wobei Oxid (1) Siliciumoxid ist und Oxid (2) ein Oxid, ausgewählt aus Aluminiumoxid, Galliumoxid, Eisenoxid, Boroxid, Titanoxid, Indiumoxid.

60. Verfahren gemäß Anspruch 58, wobei das 1-Olefinmonomer Ethylen ist.

61. Verfahren gemäß Anspruch 59, wobei das 1-Olefinmonomer Ethylen ist.

62. Verfahren gemäß Anspruch 14, wobei das Verfahren ein Verfahren ist zum Hydrogenieren einer Kohlenwasserstoffzufuhr enthaltend ungesättigte Kohlenwasserstoffe, das Verfahren umfassend Zusammenbringen der Zufuhr mit Wasserstoff unter Bedingungen, die Hydrogenierung auslösen, mit dem Katalysator.

63. Verfahren gemäß Anspruch 62, wobei der Katalysator Metalle, Salze oder Komplexe enthält, wobei das Metall ausgewählt ist aus der Gruppe Platin, Palladium, Rhodium, Iridium oder Kombinationen davon, oder der Gruppe Nickel, Molybdän, Cobalt, Wolfram, Titan, Chrom, Vanadium, Rhenium, Mangan und Kombinationen davon.

64. Verfahren zum Hydrobehandeln einer Kohlenwasserstoffzufuhr, umfassend Zusammenbringen der Zufuhr mit einem Hydrobehandlunskatalysator und Wasserstoff unter Hydrobehandlungsbedingungen, wobei der Katalysator das Molekularsieb aus Anspruch 1 umfasst.

65. Verfahren gemäß Anspruch 64, wobei der Katalysator ein Metall der Gruppe VIII oder eine solche Verbindung enthält, ein Metall der Gruppe VI oder eine solche Verbindung oder Kombinationen davon.

66. Verfahren gemäß Anspruch 64, wobei Oxid (1) Siliciumoxid ist und Oxid (2) ein Oxid, ausgewählt aus Aluminiumoxid, Galliumoxid, Eisenoxid, Boroxid, Titanoxid, Indiumoxid.

67. Verfahren gemäß Anspruch 14, wobei der Katalysator das Molekularsieb aus Anspruch 2 umfasst.

68. Verfahren gemäß Anspruch 67, wobei das Molekularsieb im Wesentlichen frei von Säure ist.

69. Verfahren gemäß Anspruch 14, 16, 17, 18, 19, 21, 24, 26, 32, 34, 38, 42 oder 67, wobei das Molekularsieb vorwiegend in der Wasserstoffform ist, wobei "vorwiegend in der Wasserstoffform" bedeutet, dass nach Kalzinierung mindestens 80% der Kationenorte im Molekularsieb durch Wasserstoffionen und/oder Ionen seltener Erden besetzt sind.

70. Verfahren zur Verringerung von Stickoxiden in einer Gaszufuhr, wobei das Verfahren umfasst Zusammenbringen der Gaszufuhr mit dem Molekularsieb aus Anspruch 1.

71. Verfahren zur Verringerung von Stickoxiden in einer Gaszufuhr in Anwesenheit von Sauerstoff, wobei das Verfahren umfasst Zusammenbringen der Gaszufuhr mit dem Molekularsieb aus Anspruch 2.

72. Verfahren gemäß Anspruch 70 oder 71, ausgeführt in der Anwesenheit von Sauerstoff.

73. Verfahren gemäß Anspruch 70 oder 71, wobei das Molekularsieb ein Metall oder Metallionen enthält, fähig zum Katalysieren der Reduktion von Stickoxiden.

74. Verfahren gemäß Anspruch 73, wobei das Metall Cobalt, Kupfer, Platin, Eisen, Chrom, Mangan, Nickel, Zink, Lanthan, Palladium, Rhodium oder Gemische davon ist.

75. Verfahren gemäß Anspruch 70 oder 71, wobei die Gaszufuhr der Abgasstrom eines Verbrennungsmotors ist.

76. Verfahren gemäß Anspruch 74, wobei die Gaszufuhr der Abgasstrom eines Verbrennungsmotors ist.

77. Verfahren zum Oxydieren von Kohlenwasserstoffen, umfassend Zusammenbringen des Kohlenwasserstoffs mit einem Oxidationsmittel in der Anwesenheit einer katalytisch wirksamen Menge des Molekularsiebs aus Anspruch 5.

78. Verfahren gemäß Anspruch 77, wobei das Verfahren ein Verfahren ist zum Epoxidieren eines Olefins, umfassend Zusammenbringen des Olefins mit Wasserstoffperoxid in der Anwesenheit des Molekularsiebs während eines Zeitraums und bei einer Temperatur, wirksam zum Epoxidieren des Olefins.

79. Verfahren gemäß Anspruch 77, wobei das Verfahren ein Verfahren ist zum Oxidieren von Cyclohexan, umfassend Zusammenbringen des Cyclohexans mit Wasserstoffperoxid in der Anwesenheit einer katalytisch wirksamen Menge des Molekularsiebs während eines Zeitraums und bei einer Temperatur, wirksam zum Oxidieren des Cyclohexans.

80. Katalytisches Oxidationsverfahren, umfassend Zusammenbringen unter Oxidationsbedingungen (1) eines Reagens, das katalytisch oxidierbar ist in der Anwesenheit von Wasserstoffperoxid, (2) wässrigen Wasserstoffperoxids und (3) einer katalytisch wirksamen Menge eines Oxidationskatalysatoren, umfassend das Molekularsieb aus Anspruch 5.

81. Verfahren gemäß Anspruch 80, wobei das elementare Molarverhältnis von Titan zu Silicium 0,005 bis 0,2 ist.

82. Verfahren gemäß Anspruch 80, wobei das elementare Molarverhältnis von Titan zu Silicium 0,01 bis 0,05 ist.

83. Verfahren gemäß Anspruch 80, wobei das oxidierbare Reagens ein Kohlenwasserstoff ist.

## Revendications

1. Tamis moléculaire ayant un rapport molaire supérieur à 15 entre (1) un oxyde d'un premier élément tétravalent et (2) un oxyde d'un élément trivalent, élément pentavalent, deuxième élément tétravalent qui est différent dudit premier élément tétravalent, ou un de leurs mélanges, et ayant après calcination les lignes de diffraction à rayons X de la Table II.
| *2-thêta* | *Espacement d (Ångström)* | *Intensité relative (%)* |
|---|---|---|
| 7,31 ± 0,15 | 12,1 | VS |
| 7,75 ± 0,15 | 11,4 | VS |
| 9,25 ± 0,15 | 9,6 | VS |
| 14,56 ± 0,15 | 6,08 | VS |
| 15,61 ± 0,15 | 5,68 | S |
| 19,60 ± 0,15 | 4,53 | S |
| 21,81 ± 0,15 | 4,07 | M |
| 22,24 ± 0,15 | 4,00 | M-S |
| 26,30 ± 0,15 | 3,39 | VS |
| 26,81 ± 0,15 | 3,33 | VS |

2. Tamis moléculaire selon la revendication 1, dans lequel le tamis moléculaire a un rapport molaire supérieur à 15 entre (1) l'oxyde de silicium et (2) un oxyde sélectionné parmi l'oxyde d'aluminium, l'oxyde de gallium, l'oxyde de fer, l'oxyde de bore, l'oxyde de titane, l'oxyde d'indium et de leurs mélanges.

3. Tamis moléculaire selon la revendication 2 dans lequel les oxydes comprennent l'oxyde de silicium et l'oxyde d'aluminium.

4. Tamis moléculaire selon la revendication 2 dans lequel les oxydes comprennent l'oxyde de silicium et l'oxyde de bore.

5. Tamis moléculaire selon la revendication 2 dans lequel les oxydes comprennent l'oxyde de silicium et l'oxyde de titane.

6. Tamis moléculaire selon la revendication 2 dans lequel le tamis moléculaire comprend essentiellement tout l'oxyde de silicium.

7. Tamis moléculaire selon la revendication 1 dans lequel ledit tamis moléculaire est surtout dans la forme hydrogénée, où « surtout dans la forme hydrogénée » signifie qu'après calcination au moins 80 % des sites cationiques dans ledit tamis moléculaire sont occupés par des ions hydrogène et/ou des ions de terres rares.

8. Tamis moléculaire selon la revendication 1 dans lequel ledit tamis moléculaire est essentiellement libre d'acidité.

9. Tamis moléculaire selon la revendication 2 dans lequel ledit tamis moléculaire est surtout dans la forme hydrogénée, où « surtout dans la forme hydrogénée » signifie qu'après calcination au moins 80 % des sites cationiques dans ledit tamis molécualire sont occupés par des ions hydrogène et/ou des ions de terres rares.

10. Tamis moléculaire selon la revendication 2 dans lequel ledit tamis moléculaire est essentiellement libre d'acidité.

11. Tamis moléculaire selon la revendication 1 ayant une composition, comme synthétisé et dans l'état anhydre, en termes de rapports molaires comme suit :
| | |
|---|---|
| YO₂/B₂O₃ | 20 à 60 |
| M_{2/n}/YO₂ | 0,01 à 0,03 |
| O/YO₂ | 0,02 à 0,05 |
| F/YO₂ | 0 à 0,10 |
où Y est le silicium, M est un cation de métal alcalin, cation de métal alcalino-terreux ou un de leurs mélanges ; n est la valence de M ; F est le fluor et Q est un cation N,N'-diisopropylimidazolium.

12. Procédé pour la préparation d'un tamis moléculaire de la revendication 4, ledit procédé comprenant contacter dans des conditions de cristallisation des sources desdits oxydes, un agent de direction de structure comprenant un cation N,N'-diisopropylimidazolium et des ions fluorure.

13. Procédé selon la revendication 12 dans lequel ledit tamis moléculaire est préparé à partir d'un mélange réactionnel comprenant, en termes de rapports molaires :
| | |
|---|---|
| YO₂/B₂O₃ | 5 à 60 |
| OH-/YO₂ | 0,10 à 0,50 |
| O/YO₂ | 0,05 à 0,50 |
| M_{2/n}/YO₂ | 0,02 à 0,40 |
| H₂O/YO₂ | 30 à 80 |
| F/YO₂ | 0 à 0,50 |
où Y est le silicium, M est un cation de métal alcalin, cation de métal alcalino-terreux ou un de leurs mélanges ; n est la valence de M ; F est le fluor et Q est un cation N,N'-diisopropylimidazolium.

14. Procédé pour convertir des hydrocarbures comprenant contacter une alimentation hydrocarbonée dans des conditions de conversion d'hydrocarbures avec un catalyseur comprenant le tamis moléculaire de la revendication 1 ou de la revendication 2.

15. Procédé selon la revendication 14 dans lequel le tamis moléculaire est essentiellement libre d'acidité.

16. Procédé selon la revendication 14 dans lequel le procédé est un procédé d'hydrocraquage comprenant contacter le catalyseur avec une alimentation hydrocarbonée dans des conditions d'hydrocraquage.

17. Procédé selon la revendication 14 dans lequel le procédé est un procédé de déparaffinage comprenant contacter le catalyseur avec une alimentation hydrocarbonée dans des conditions de déparaffinage.

18. Procédé selon la revendication 14 dans lequel le procédé est un procédé pour améliorer l'indice de viscosité d'un produit déparaffiné d'alimentations hydrocarbonées cireuses comprenant contacter le catalyseur avec une alimentation hydrocarbonée cireuse dans des conditions de déparaffinage d'isomérisation.

19. Procédé selon la revendication 14 dans lequel le procédé est un procédé pour produire une huile lubrifiante C₂₀₊ à partir d'une alimentation d'oléfines C₂₀₊ comprenant isomériser ladite alimentation d'oléfines dans des conditions d'isomérisation sur le catalyseur.

20. Procédé selon la revendication 19 dans lequel le catalyseur comprend en plus au moins un métal du groupe VIII.

21. Procédé selon la revendication 14 dans lequel le procédé est un procédé pour le déparaffinage catalytique d'une alimentation d'huile hydrocarbonée bouillant au-dessus de 350°F (177°C) et contenant des hydrocarbures à chaînes droites et à chaînes légèrement branchées comprenant contacter ladite alimentation d'huile hydrocarbonée dans la présence de gaz d'hydrogène ajouté à une pression d'hydrogène de 15 à 3000 psi (0,103 à 20,7 MPa) dans des conditions de déparaffinage avec le catalyseur.

22. Procédé selon la revendication 21 dans lequel le catalyseur comprend en plus au moins un métal du groupe VIII.

23. Procédé selon la revendication 21 dans lequel le catalyseur comprend un catalyseur en couches comprenant une première couche comprenant le tamis moléculaire et au moins un métal du groupe VIII, et une deuxième couche comprenant un zéolite d'aluminosilicate qui est davantage sélectif pour les formes que le tamis moléculaire de ladite première couche.

24. Procédé selon la revendication 14 dans lequel le procédé est un procédé pour préparer une huile lubrifiante qui comprend :
hydrocraquage dans une zone d'hydrocraquage d'une alimentation hdyrocarbonée pour obtenir un effluent comprenant une huile hydrocraquée ; et
le déparaffinage catalytique dudit effluent comprenant de l'huile hydrocraquée à une température d'au moins 400°F (204°C) et à une pression entre 15 psig et 3000 psig (0,103 à 20,7 MPa pression manométrique) dans la présence de gaz d'hydrogène ajouté avec le catalyseur.

25. Procédé selon la revendication 24 dans lequel le catalyseur comprend en plus au moins un métal du groupe VIII.

26. Procédé selon la revendication 14 dans lequel le procédé est un procédé pour le déparaffinage d'isomérisation d'un raffinat comprenant contacter ledit raffinat dans la présence d'hydrogène ajouté dans de condition de déparaffinage d'isomérisation avec le catalyseur.

27. Procédé selon la revendication 26 dans lequel le catalyseur comprend en plus au moins un métal du groupe VIII.

28. Procédé selon la revendication 26 dans lequel le raffinat est le bright stock.

29. Procédé selon la revendication 14 dans lequel le procédé est un procédé pour augmenter l'octane d'une alimentation hydrocarbonée pour produire un produit ayant un contenu en aromatiques augmenté comprenant contacter une alimentation hydrocarbonée qui comprend des hydrocarbures normaux et légèrement branchés ayant un domaine d'ébullition au-dessus de 40°C et de moins de 200°C dans des conditions de conversion aromatique avec le catalyseur.

30. Procédé selon la revendication 29 dans lequel le tamis moléculaire est essentiellement libre d'acidité.

31. Procédé selon la revendication 29 dans lequel le catalyseur comprend en plus au moins un métal du groupe VIII.

32. Procédé selon la revendication 14 dans lequel le procédé est un procédé de craquage catalytique comprenant contacter une alimentation hydrocarbonée dans une zone de réaction dans des conditions de craquage catalytique dans l'absence d'hydrogène ajouté avec le catalyseur.

33. Procédé selon la revendication 32 dans lequel le catalyseur comprend en plus une composante de craquage cristalline à grandes pores.

34. Procédé selon la revendication 14 dans lequel le procédé est un procédé d'isomérisation pour isomériser des hydrocarbures C₄ à C₇, comprenant contacter une alimentation ayant des hydrocarbures C₄ à C₇ normaux et légèrement branchés dans des conditions d'isomérisation avec le catalyseur.

35. Procédé selon la revendication 34 dans lequel le tamis moléculaire a été imprégné avec au moins un métal du groupe VIII.

36. Procédé selon la revendication 35 dans lequel le catalyseur a été calciné dans un mélange vapeur/air à une température élevée après l'imprégnation avec le métal du groupe VIII.

37. Procédé selon la revendication 35 dans lequel le métal du groupe VIII est le platine.

38. Procédé selon la revendication 14 dans lequel le procédé est un procédé pour l'alkylation d'un hydrocarbure aromatique qui comprend contacter dans des conditions d'alkylation au moins un excès molaire d'un hydrocarbure aromatique avec une oléfine C₂ à C₂₀ dans des conditions au moins partiellement de phase liquide et dans la présence du catalyseur.

39. Procédé selon la revendication 38 dans lequel l'oléfine est une oléfine C₂ à C₄.

40. Procédé selon la revendication 39 dans lequel l'hydrocarbure aromatique et l'oléfine sont présents dans un rapport molaire de 4:1 à 20:1, respectivement.

41. Procédé selon la revendication 39 dans lequel l'hydrocarbure aromatique est sélectionné parmi le groupe constitué du benzène, du toluène, de l'éthylbenzène, du xylène, de la naphtaline, des dérivés de la naphtaline, de la diméthylnaphtaline ou de leurs mélanges.

42. Procédé selon la revendication 14 dans lequel le procédé est un procédé pour la transalkylation d'un hydrocarbure aromatique qui comprend contacter dans des conditions de transalkylation un hydrocarbure aromatique avec un hydrocarbure polyalkylaromatique dans des conditions au moins partiellement de phase liquide et dans la présence du catalyseur.

43. Procédé selon la revendication 42 dans lequel l'hydrocarbure aromatique et l'hydrocarbure polyalkylaromatique sont présents dans un rapport molaire entre 1:1 et 25:1, respectivement.

44. Procédé selon la revendication 42 dans lequel l'hydrocarbure aromatique est sélectionné parmi le groupe constitué du benzène, du toluène, de l'éthylbenzène, du xylène, ou de leurs mélanges.

45. Procédé selon la revendication 42 dans lequel l'hydrocarbure polyalkylaromatique est un dialkylbenzène.

46. Procédé selon la revendication 14 dans lequel le procédé est un procédé de conversion de paraffines en aromates qui comprend contacter des paraffines dans des conditions qui causent des paraffines de convertir en aromates avec un catalyseur comprenant le tamis moléculaire et du gallium, du zinc ou un composé de gallium ou de zinc.

47. Procédé selon la revendication 14 dans lequel le procédé est un procédé pour isomériser des oléfines comprenant contacter ladite oléfine dans des conditions qui causent l'isomérisation de l'oléfine avec le catalyseur.

48. Procédé selon la revendication 14 dans lequel le procédé est un procédé pour isomériser une alimentation d'isomérisation comprenant une alimentation de C₈ aromatique d'isomères du xylène ou de mélanges d'isomères du xylène et de l'éthylbenzène, où un rapport plus proche d'équilibre d'ortho, méta et paraxylènes est obtenu, ledit procédé comprenant contacter ladite alimentation dans des conditions d'isomérisation avec ledit catalyseur.

49. Procédé selon la revendication 14 dans lequel le procédé est un procédé pour oligomériser des oléfines comprenant contacter une alimentation d'oléfines dans des conditions d'oligomérisation avec le catalyseur.

50. Procédé selon la revendication 14 dans lequel le procédé est un procédé pour convertir des hydrocarbures oxygénés comprenant contacter lesdits hydrocarbures oxygénés dans des conditions pour produire des produits liquides avec le catalyseur.

51. Procédé selon la revendication 50 dans lequel l'hydrocarbure oxygéné est un alcool inférieur ayant 1 à 10 atomes de carbone.

52. Procédé selon la revendication 51 dans lequel l'alcool inférieur est le méthanol.

53. Procédé selon la revendication 14 dans lequel le procédé est un procédé pour la production d'hydrocarbures à poids moléculaire plus élevé à partir d'hydrocarbures à poids moléculaire moins élevés comprenant les étapes de :
(a) introduire dans une zone de réaction un gaz contenant des hydrocarbures à poids moléculaires moins élevés et contacter ledit gaz dans ladite zone dans des conditions de synthèse d'hydrocarbure C₂₊ avec le catalyseur et un métal ou composé métallique apte à convertir l'hydrocarbure à poids moléculaire moins élevé en un hydrocarbure à poids moléculaire plus élevé ; et
(b) enlever de ladite zone de réaction une alimentation d'hydrocarbure à poids moléculaire plus élevé.

54. Procédé selon la revendication 53 dans lequel le métal ou composé métallique comprend un métal ou composé métallique des lanthanides ou actinides.

55. Procédé selon la revendication 53 dans lequel l'hydrocarbure à poids moléculaire moins élevé est le méthane.

56. Composition catalytique pour promouvoir la polymérisation d'1-oléfines, ladite composition comprenant
(A) le tamis moléculaire de la revendication 1 ; et
(B) un composé d'organotitane ou d'organochrome.

57. Composition catalytique selon la revendication 56 dans lequel l'oxyde (1) est l'oxyde de silicium et l'oxyde (2) est un oxyde sélectionné parmi l'oxyde d'aluminium, l'oxyde de gallium, l'oxyde de fer, l'oxyde de bore, l'oxyde de titane, l'oxyde d'indium.

58. Procédé selon la revendication 14 dans lequel le procédé est un procédé de polymérisation d'1-oléfines, ledit procédé comprenant contacter du monomère 1-oléfinique avec une quantité catalytiquement effective de la composition catalytique selon la revendication 56 dans des conditions de polymérisation qui comprennent une température et une pression convenables pour initier et promouvoir une réaction de polymérisation.

59. Procédé selon la revendication 58 dans lequel l'oxyde (1) est l'oxyde de silicium et l'oxyde (2) est un oxyde sélectionné parmi l'oxyde d'aluminium, l'oxyde de gallium, l'oxyde de fer, l'oxyde de bore, l'oxyde de titane, l'oxyde d'indium.

60. Procédé selon la revendication 58 dans lequel le monomère 1-oléfinique est l'éthylène.

61. Procédé selon la revendication 59 dans lequel le monomère 1-oléfinique est l'éthylène.

62. Procédé selon la revendication 14 dans lequel le procédé est un procédé pour hydrogéner une alimentation hydrocarbonée contenant des hydrocarbures insaturés, le procédé comprenant contacter l'alimentation avec de l'hydrogène dans des conditions qui causent une hydrogénation avec le catalyseur.

63. Procédé selon la revendication 62 dans lequel le catalyseur contient des métaux, sels ou complexes, le métal étant sélectionné parmi le groupe constitué du platine, du palladium, du rhodium, de l'iridium ou de leur combinaisons, ou le groupe constitué du nickel, du molybdène, du cobalt, du tungstène, du titane, du chrome, du vanadium, du rhénium, du manganèse et de leurs combinaisons.

64. Procédé pour hydrotraiter une alimentation hydrocarbonée comprenant contacter l'alimentation avec un catalyseur d'hydrotraitement et de l'hydrogène dans des conditions d'hydrotraitement, dans lequel le catalyseur comprend le tamis moléculaire de la revendication 1.

65. Procédé selon la revendication 64 dans lequel le catalyseur contient un métal ou composé du groupe VIII, un métal ou composé du groupe VI ou leurs combinaisons.

66. Procédé selon la revendication 64 dans lequel l'oxyde (1) est l'oxyde de silicium et l'oxyde (2) est un oxyde sélectionné parmi l'oxyde d'aluminium, l'oxyde de gallium, l'oxyde de fer, l'oxyde de bore, l'oxyde de titane, l'oxyde d'indium.

67. Procédé selon la revendication 14 dans lequel le catalyseur comprend le tamis moléculaire de la revendication 2.

68. Procédé selon la revendication 67 dans lequel le tamis moléculaire est essentiellement libre d'acidité.

69. Procédé selon la revendication 14, 16, 17, 18, 19, 21, 24, 26, 32, 34, 38, 42 ou 67 dans lequel le tamis moléculaire est surtout dans la forme hydrogénée, où « surtout dans la forme hydrogénée » signifie qu'après calcination au moins 80 % des sites cationiques dans ledit tamis moléculaire sont occupés par des ions hydrogène et/ou des ions de terres rares.

70. Procédé pour la réduction d'oxydes d'azote contenus dans une alimentation de gaz dans lequel ledit procédé comprend contacter l'alimentation de gaz avec le tamis moléculaire de la revendication 1.

71. Procédé pour la réduction d'oxydes d'azote contenus dans une alimentation de gaz dans la présence d'oxygène dans lequel ledit procédé comprend contacter l'alimentation de gaz avec le tamis moléculaire de la revendication 2.

72. Procédé selon la revendication 70 ou 71 conduit dans la présence d'oxygène.

73. Procédé selon la revendication 70 ou 71 dans lequel ledit tamis moléculaire contient un métal ou des ions métalliques capables de catalyser I réduction des oxydes d'azote.

74. Procédé selon la revendication 73 dans lequel le métal est le cobalt, le cuivre, le platine, le fer, le chrome, le manganèse, le nickel, le zinc, le lanthane, le palladium, le rhodium ou leurs mélanges.

75. Procédé selon la revendication 70 ou 71 dans lequel l'alimentation de gaz est l'alimentation d'échappement d'un moteur à combustion interne.

76. Procédé selon la revendication 74 dans lequel l'alimentation de gaz est l'alimentation d'échappement d'un moteur à combustion interne.

77. Procédé pour l'oxydation d'hydrocarbures comprenant contacter ledit hydrocarbure avec un agent d'oxydation dans la présence d'une quantité catalytiquement effective du tamis moléculaire de la revendication 5.

78. Procédé selon la revendication 77 dans lequel le procédé est un procédé d'époxydation d'une oléfine comprenant contacter ladite oléfine avec du peroxyde d'hydrogène dans la présence du tamis moléculaire pendant une durée et à une température effectives pour époxyder ladite oléfine.

79. Procédé selon la revendication 77 dans lequel le procédé est un procédé pour oxyder du cyclohexane comprenant contacter ledit cyclohexane avec du peroxyde d'hydrogène dans la présence d'une quantité catalytiquement effective du tamis moléculaire pendant une durée et à une température effectives pour oxyder le cyclohexane.

80. Procédé d'oxydation catalytique comprenant contacter dans des conditions d'oxydation (1) un réactif qui est catalytiquement oxydable dans la présence de peroxyde d'hydrogène, (2) du peroxyde d'hydrogène aqueux et (3) une quantité catalytiquement effective d'un catalyseur d'oxydation comprenant le tamis moléculaire de la revendication 5.

81. Procédé selon la revendication 80 dans lequel le rapport molaire élémentaire entre titane et silicium est 0,005 à 0,2.

82. Procédé selon la revendication 80 dans lequel le rapport molaire élémentaire entre titane et silicium est 0,01 à 0,05.

83. Procédé selon la revendication 80 dans lequel le réactif oxydable est un hydrocarbure.
